(19) Europäisches Patentamt
European Patent Office
Office européen des brevets

(11) EP 4 759 792 A1

(12) **EUROPEAN PATENT APPLICATION**

(43) Date of publication:
**17.06.2026 Bulletin 2026/25**

(21) Application number: **24315572.8**

(22) Date of filing: **12.12.2024**

(51) International Patent Classification (IPC):
***C07C 5/333*** *(2006.01)* ***C07C 11/06*** *(2006.01)*
***C10G 3/00*** *(2006.01)* ***C25B 3/00*** *(2021.01)*

(52) Cooperative Patent Classification (CPC):
(C-Sets available)
**C07C 5/3337; C10G 3/50; C10G 11/02; C10G 11/04; C25B 3/03; C25B 3/25; C25B 9/23; C25B 11/077; C25B 13/07;** C07C 2521/04; C07C 2523/10; C07C 2523/14; C07C 2523/42; C07C 2523/63; C10G 2300/1014 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **TotalEnergies OneTech**
**92400 Courbevoie (FR)**

(72) Inventor: **Grand, Julien**
**7181 Seneffe (BE)**

(74) Representative: **Hensenne, Peter**
**Patent42**
**5, rue Dicks**
**4081 Esch-sur-Alzette (LU)**

(54) **GREEN PROPANE DEHYDROGENATION PROCESS**

(57) The disclosure concerns a propane dehydrogenation process, remarkable in that it comprises the steps of (a) providing a feed (1) comprising one or more vegetable oils; (b) providing an hydrogen stream (3) and performing an hydrogenation reaction on said feed (1) to generate at least one effluent (6) comprising propane and one or more fatty acids; (c) separating propane from said one or more fatty acids comprised within said at least effluent (6) generated at step (b) to generate a stream (13) comprising propane; (d) providing at least one proton-conducting catalytic membrane, each proton-conducting catalytic membrane comprising at least one dehydrogenation catalyst; (e) feeding to said one or more proton-conducting catalytic membranes under propane dehydrogenation conditions the stream generated at step (c); and (f) recovering a first effluent (19) comprising at least propylene. The disclosure also concerns an installation for carrying out said propane dehydrogenation process thereof.

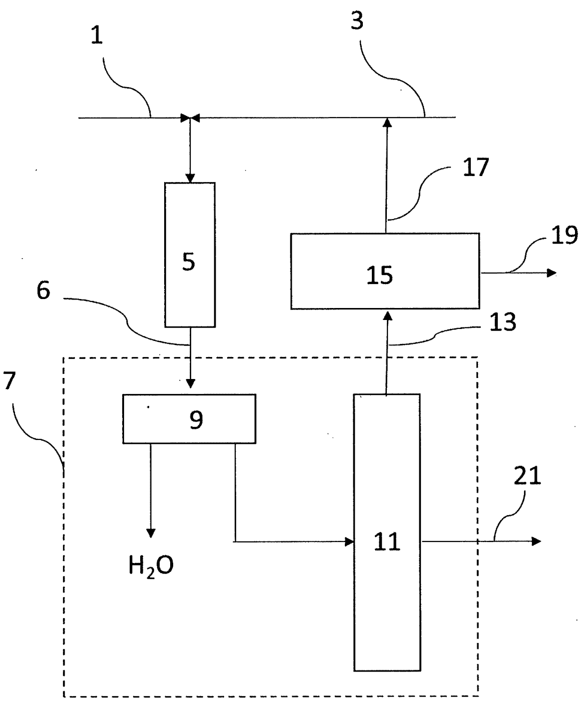


Fig. 1



Processed by Luminess, 75001 PARIS (FR)

(Cont. next page)

(52) Cooperative Patent Classification (CPC): (Cont.)

C-Sets
**C07C 5/3337, C07C 11/06**

## Description

### Technical field

The present disclosure relates to a green propane dehydrogenation process.

### Technical background

Propylene is a valuable starting material to make plastics, such as polypropylene, or sustainable fuels, such as sustainable aviation fuels (SAF). To produce propylene from propane, a dehydrogenation reaction must be carried out. Dehydrogenation reactions are generally carried out without hydrogen extraction. Thus, industrial processes are generally limited to about 50% conversion. In addition, such reactions lead to significant and fast coking of the catalyst. Industrial processes require therefore continuous decoking (with $H_2$ or water). $H_2$ produced must be separated from the product stream after the reaction.

A solution was brought by EP2534721 and/or by WO2011098525 to use a tubular reactor comprising a first zone comprising a dehydrogenation catalyst and a second zone separated from said first zone by a proton-conducting membrane comprising forming a layered structure of a porous and non-porous mixed metal oxide as a support and a dehydrogenation catalyst that is deposited on the membrane and that can adhere to it or freely lie on it. Similar proton-conducting ceramic membrane are described in WO2014187978.

In US20200248321, a membrane reactor for conducting the dehydrogenation of alkanes to alkenes presents one or more tubes lined with one or more reactive ceramic membranes or layered throughout the one or more tubes. The membrane reactor allows a heated feed of alkanes to come into contact with the one or more ceramic membranes, wherein an alkane would react therewith, allowing alkenes to continue to flow through the one or more tubes and out of the system in reactor effluent feed, while hydrogen generated from the reaction of the alkane with the membrane would pass through the membrane and be physically separated.

However, it appears that the manufacturing of defect-free straight tubes is a critical issue for further development of the dense ion-conducting membrane propane-to-propylene on-purpose technology. Defects in the membrane enhance coke formation with the fatal result that the membrane is destroyed. The fragility of the membranes is also an issue.

Another configuration exists, in which the dehydrogenation catalyst is not in contact with the hydrogen transport membrane. Thus, WO2015052297 refers to a process for preparing aromatic hydrocarbon from alkanes, wherein alkanes are contacted with a dehydrogenation catalyst in presence of hydrogen to limit coking. The hydrogen is then extracted from the reactor with a hydrogen transport membrane made for example of mixed metal oxides.

Propane, however, is mainly originating from petroleum resources. This is problematic, because society nowadays needs sustainable plastics and sustainable fuels and having recourse to petroleum resources is less and less acceptable.

### Summary

According to a **first aspect,** the disclosure relates to a propane dehydrogenation (PDH) process, said process is remarkable in that it comprises the following steps:

a) providing a feed comprising one or more vegetable oils;
b) providing an hydrogen stream and performing an hydrogenation reaction on said feed to generate at least one effluent comprising propane and one or more fatty acids;
c) separating propane from said one or more fatty acids comprised within said at least one effluent generated at step (b) to generate a stream comprising propane;
d) providing at least one proton-conducting catalytic membrane, each proton-conducting catalytic membrane comprising at least one dehydrogenation catalyst;
e) feeding to said one or more proton-conducting catalytic membranes under propane dehydrogenation conditions the stream generated at step (c);
f) recovering a first effluent comprising at least propylene;
g) optionally, recovering a second effluent comprising hydrogen.

Surprisingly, the PDH process of the present disclosure is about a solution to the generation of green propylene, and optionally green hydrogen. The PDH process of the present disclosure has the advantage of using abundant natural resources, such as one or more vegetable oils. This is therefore a sustainable process for producing in industrial quantities propylene, which is a raw material used in many downstream processes. This subsequently can bring an answer to the current need of sustainable plastics and sustainable fuels.

Advantageously, the feed comprising one or more vegetable oils comprises one or more triglycerides.

For example, each triglyceride has three aliphatic chains, and the process is remarkable in that at least two of the three aliphatic chains are identical and have a number of carbons ranging between 7 and 22, or between 10 and 20, or between 16 and 20, or between 16 and 18. With preference, each triglyceride has three aliphatic chains, and the process is remarkable in that the three aliphatic chains are identical and have a number of carbons ranging between 7 and 22; or between 10 and 20, or between 16 and 20, or between 16 and 18.

Advantageously, the hydrogenation reaction performed in step (b) is carried out at a flow of at most 200 T/hour, or at most 190 T/hour, or at most 180 T/hour, or at most 170 T/hour, or at most 160 T/hour, or at most 150 T/hour, or at most 140 T/hour, or at most 130 T/hour, or at most 120 T/hour, or at most 110 T/hour, or at most 100 T/hour, or of at most 90 T/hour, or of at most 80 T/hour.

Advantageously, step (g) is carried out, and the process is remarkable in that the second effluent recovered is used at least partially, or fully, in the hydrogenation reaction performed at step (b).

With preference, the process is remarkable in that the part of the second effluent comprising hydrogen recovered is used at least partially in the hydrogenation reaction performed at step (b), amounts to between 3 wt.% and 10 wt.% of the total weight of the hydrogen stream, preferably between 3.1 wt.% and 6.0 wt.%, or between 3.2 w.% and 5.5 wt.%, or between 3.3 wt.% and 5.5 wt.%, or between 3.3 wt.% and 5.3 wt.%, or between 3.4 wt.% and 5.0 wt.%.

Advantageously, step (g) is carried out, and the process is remarkable in that the second effluent recovered is used at least partially, or fully, during a hydrotreating process of the feed provided at step (a). For example, said hydrotreating process comprises the hydrogenation reeaction of the feed performed at step (b).

Advantageously, step (g) is carried out, and the process is remarkable in that the second effluent recovered is used at least partially, or fully, during a hydroizomerisation process of one or more hydrotreated oils.

For example, said one or more hydrotreated oils are generated from a hydrotreating process of the feed and said hydrotreating process comprises the hydrogenation of the feed performed at step (b).

Advantageously, each proton-conducting catalytic membrane comprises an anode, an electrolyte layer disposed on top of the anode and a porous cathode disposed on top of the electrolyte layer, wherein said at least one dehydrogenation catalyst is comprised within the anode.

Advantageously, the anode comprises one or more channels, each of said channels comprising said at least one dehydrogenation catalyst.

With preference, said one or more channels are made in copper or steel.

Advantageously, said one or more channels are flared depth channels or rectangular channels. With preference, said one or more channels are flared depth channels.

Advantageously, the one or more dehydrogenation catalysts comprise one or more metal-based catalysts, one or more metal oxide-based catalysts, one or more zeolites, one or more transition metal carbides, one or more transition metal nitrides, one or more carbon nanotubes, or any mixtures thereof.

Advantageously, and alternatively, said one or more proton-conducting catalytic membranes are selected from one or more palladium membranes, one or more palladium-silver alloy membranes, one or more silica/alumina membranes, one or more zeolite-based membranes, one or more ceramic membranes, one or more ceramic electrolytic membranes, one or more polymeric membranes or any combinations thereof.

Advantageously, the propane dehydrogenation conditions of step (e) comprise a temperature ranging between 425°C and 575°C; preferably between 430°C and 570°C, more preferably between 435°C and 565°C.

For example, the propane dehydrogenation conditions comprise a space velocity of at least 150 Nml/h/g, or of at least 200 Nml/h/g, or of at least 250 Nml/h/g, or of at least 300 Nml/h/g, or of at least 350 Nml/h/g, or of at least 400 Nml/h/g, or of at least 450 Nml/h/g, or of at least 500 Nml/h/g. For example, the propane dehydrogenation conditions comprise a space velocity ranging between 150 Nml/h/g and 1000 Nml/h/g, or between 200 Nml/h/g and 1000 Nml/h/g, or between 250 Nml/h/g and 1000 Nml/h/g, or between 300 Nml/h/g and 1000 Nml/h/g, or between 350 Nml/h/g and 1000 Nml/h/g, or between 400 Nml/h/g and 1000 Nml/h/g, or between 450 Nml/h/g and 1000 Nml/h/g, or between 500 Nml/h/g and 1000 Nml/h/g.

In a preferred embodiment, the stream comprising propane generated at step (c) further comprises steam, preferably in an amount ranging between 1 vol.% and 15 vol.% based on the total volume of the stream comprising propane generated at step (c); more preferably ranging between 2 vol.% and 10 vol.%.

For example, the process further comprises a step of providing steam into the stream comprising propane generated at step (c). Said step of providing steam is performed continuously or in a pulsed manner (namely at intervals), preferably in a pulsed manner.

Advantageously, in said preferred embodiment, the one or more proton-conducting catalytic membranes provided at step (d) are one or more co-ionic catalytic membranes.

According to a second aspect, the disclosure relates to an Installation for performing a propane dehydrogenation process, said installation comprising at least one dehydrogenation reactor comprising at least one dehydrogenation catalyst and is remarkable in that at least one hydrotreating unit is placed upstream of said one or more dehydrogenation

reactors, with a propane-separation unit being placed between said one or more hydrotreating unit and said one or more dehydrogenation reactors, said one or more hydrotreating unit, one or more propane-separation unit and said one or more dehydrogenation reactors being in fluidic connection.

With preference, said propane-separation unit comprises a separator and/or a distillation column.

**Description of the figures**

- Figure 1: Installation for carrying out the propane dehydrogenation process of the present disclosure.
- Figure 2: Scheme of the three layers forming the proton-conducting catalytic membrane of the present disclosure.
- Figure 3: Scanning electron microscopy image of the proton-conducting catalytic membrane of the present disclosure.
- Figure 4: Scheme of a propane dehydrogenation (PDH) process carried out with the proton-conducting catalytic membrane of the present disclosure.
- Figure 5: Scheme of a rectangular channel arranged in the electroconductive layer (i.e., the anode) of a dehydrogenation reactor of the present disclosure.
- Figure 6: Scheme of a flared depth channel arranged in the electroconductive layer (i.e., the anode) of a dehydrogenation reactor of the present disclosure, the flared depth channel having a surface area of the outlet larger than the surface area of the inlet.
- Figure 7: Representation of the mechanism when a co-ionic catalytic membrane is used.
- Figure 8: Arrangement of 4 proton-conducting catalytic membranes according to the present disclosure in a stacking forming a dehydrogenation reaction with 4 membranes working in parallel.
- Figure 9: Arrangement of 20 proton-conducting catalytic membranes according to the present disclosure in a stacking forming a dehydrogenation reaction with 20 membranes working in parallel.
- Figure 10: Electrochemical Impedance Spectroscopy (EIS) measurements of the electrolyte used in the present disclosure. Z' and Z" are respectively the real and the imaginary part of the impedance, each part corresponding to the two phases of the electrical impedance. The real part corresponds to the resistance R while the imaginary part corresponds to the reactance X.
- Figure 11: Zoom-in of figure 10 at the zone of the intersect of the curves with the abscissa.
- Figure 12: Conductivity measurement of the electrolytes used in the present disclosure. The electrolytes **1A** and **1B** have been pre-calcined and then sintered at 700°C while the electrolyte **2A** has been sintered at 700°C.
- Figure 13: Evolution of the electrical potential in function of the area specific resistance in the proton-conducting catalytic membrane according to the disclosure.
- Figure 14: Conversion of propane into propene in function of the $H_2$ extraction ratio with respect to the temperature.
- Figure 15: Conversion of propane into propene in function of the $H_2$ extraction ratio with respect to the pressure.
- Figure 16: Propane conversion and propylene selectivity at a space velocity of 150 Nml/h/g as a function of the temperature and the hydrogen extraction ratio.
- Figure 17: Propane conversion and propylene selectivity at a space velocity of 750 Nml/h/g as a function of the temperature and the hydrogen extraction ratio.
- Figure 18: Simulation by computational fluid dynamics (CFD) of the coke formation in function of the temperature within the proton-conducting catalytic membranes of the present disclosure.
- Figure 19: Representation of a flared depth channel in accordance with the present disclosure.
- Figure 20: Evolution of the amount of coke in function of the reactor length in the absence of steam in the flared depth channel of figure 19.
- Figure 21: Evolution of the amount of coke in function of the reactor length when water is co-fed with the propane and when the reactor employs a proton-conducting catalytic membrane in accordance with the disclosure in the flared depth channel of figure 19.
- Figure 22: Evolution of the amount of coke in function of the reactor length when water is co-fed with the propane and when the reactor employs a co-ionic catalytic membrane in accordance with the disclosure in the flared depth channel of figure 19.
- Figure 23: Propylene yield as a function of the time on stream (TOS) comprising water at a molar fraction of 2.7 %.
- Figure 24: Propylene yield as a function of the time on stream (TOS) comprising water at a molar fraction of 5.4 %.
- Figure 25: Propylene yield as a function of the time on stream (TOS) comprising water at a molar fraction of 7.9 %.
- Figure 26: Proton-conducting catalytic membrane in which the anode is made of copper, the membrane being incorporated within a steel housing.
- Figure 27: Proton-conducting catalytic membrane in which the anode is made of stainless steel.
- Figure 28: Photograph of the proton-conducting catalytic membrane in which the anode is made of stainless steel.

## Detailed description

For the disclosure, the following definitions are given:
The terms "comprising", "comprises" and "comprised of' as used herein are synonymous with "including", "includes" or "containing", "contains", and are inclusive or open-ended and do not' exclude additional, non-recited members, elements or method steps. The terms "comprising", "comprises" and "comprised of' also include the term "consisting of".

The recitation of numerical ranges by endpoints includes all integer numbers and, where appropriate, fractions subsumed within that range (e.g., 1 to 5 can include 1, 2, 3, 4, 5 when referring to, for example, a number of elements, and can also include 1.5, 2, 2.75 and 3.80, when referring to, for example, measurements). The recitation of endpoints also includes the recited endpoint values themselves (e.g., from 1.0 to 5.0 includes both 1.0 and 5.0). Any numerical range recited herein is intended to include all sub-ranges subsumed therein.

Zeolite codes (e.g., CHA...) are defined according to the "Atlas of Zeolite Framework Types", 6th revised edition, 2007, Elsevier, to which the present application also makes reference.

As used herein, the term "C# hydrocarbons", wherein "#" is a positive integer, is meant to describe all hydrocarbons having # carbon atoms. C# hydrocarbons are sometimes indicated as just C#.

The symbol "=" in the term "C#= hydrocarbon" indicates that the hydrocarbon concerned is an olefin or an alkene, and the notation "=" symbolises the carbon-carbon double bond. For instance, "C6=" stands for "C6 olefin", or for "olefins comprising 6 carbon atoms".

The term "steam" is used to refer to water in the gas phase, which is formed when water boils.

The term "transition metal" refers to an element whose atom has a partially filled d sub-shell, or which can give rise to cations with an incomplete d sub-shell (IUPAC definition). According to this definition, the transition metals are Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg, Ac, Rf, Db, Sg, Bh, Hs, Mt, Ds, Rg, and Cn.

The metals Ga, In, Sn, Ti, Pb and Bi are considered "post-transition" metals.

The metals Au, Ag, Ru, Rh, Pd, Os, Ir and Pt show outstanding oxidation resistance and are considered "noble" metals. Other metals can be considered "non-noble" metals.

The term "alkali metal" refers to an element classified as an element from group 1 of the periodic table of elements (or group IA), excluding hydrogen. According to this definition, the alkali metals are Li, Na, K, Rb, Cs and Fr.

The term "alkaline earth metal" refers to an element classified as an element from group 2 of the periodic table of elements (or group IIA). According to this definition, the alkaline earth metals are Be, Mg, Ca, Sr, Ba and Ra.

The term "rare earth elements" refer to the fifteen lanthanides, as well as scandium and yttrium. The 17 rare-earth elements are cerium (Ce), dysprosium (Dy), erbium (Er), europium (Eu), gadolinium (Gd), holmium (Ho), lanthanum (La), lutetium (Lu), neodymium (Nd), praseodymium (Pr), promethium (Pm), samarium (Sm), scandium (Sc), terbium (Tb), thulium (Tm), ytterbium (Yb), and yttrium (Y).

The name "glyceride" encompasses chemical compounds which are esters derived from glycerol and fatty acids. In particular, "triglyceride" relates to a chemical compound which is an ester of glycerol and three fatty acids. The triglyceride is chemically represented by the below formula, wherein R represent an aliphatic chain. Each aliphatic chain R can have a number of carbons ranging between 7 and 22.

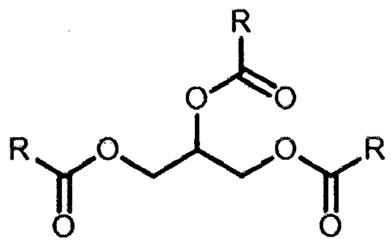

The space velocity (Nml/h/g) is measured in term of the volumetric flow rate (Nml/h) of the reactant at 0°C and 1.01 bar per gram of catalyst ($g^{-1}$). The volumetric flow rate of a fluid is expressed in Nml/h ("N" stands for "Normalized"), which corresponds to 1 $cm^3_{NTP}$/h.

The feed flow (T/h) is measured by a flowmeter and corresponds to the amount of ton per hour that is flowing.

The particular features, structures, characteristics or embodiments may be combined in any suitable manner, as would be apparent to a person skilled in the art from this disclosure, in one or more embodiments.

The present disclosure relates to a propane dehydrogenation (PDH) process remarkable in that it comprises the following steps:

a) providing a feed 1 comprising one or more vegetable oils;

b) providing an hydrogen stream 3 and performing an hydrogenation reaction on said feed 1 to generate at least one effluent 6 comprising propane and one or more fatty acids;

c) separating propane from said one or more fatty acids comprised within said at least one effluent 6 generated at step (b) to generate a stream 13 comprising propane;

d) providing at least one proton-conducting catalytic membrane 15, each proton-conducting catalytic membrane comprising at least one dehydrogenation catalyst;

e) feeding to said one or more proton-conducting catalytic membranes 15 under propane dehydrogenation conditions the stream 13 generated at step (c);

f) recovering a first effluent 19 comprising at least propylene.

Optionally, a step (g) of recovering a second effluent 17 comprising hydrogen can be carried out.

Since the one or more vegetable oils comprises generally a large quantity of one or more triglycerides, the feed 1 comprising one or more vegetable oils comprises one or more triglycerides. It is also possible to carry out the PDH process of the presnt disclosure on a feed 1 of triglycerides.

The PDH process of the present disclosure is thus a solution to enhance the obtention of sustainable propylene, since the stream 13 of propane that is brought to one or more proton-conducting catalytic membranes is coming from an effluent of an hydrogenation reaction of a feed 1 comprising, or consisting of, one or more vegetable oils or one or more triglycerides, which are abundant natural products.

Figure 1 describes a feed 1 comprising, or consisting of, one or more vegetable oils, or one or more triglyceride oils, that are directed into at least one hydrotreating unit 5, in order to be subjected to a hydrogenation reaction. Subsequently, an effluent 6 comprising propane and one or more fatty acids is generated. Because of the origin of the feed 1, the propane in the effluent 6 can be called green propane. The effluent 6 comprising propane and one or more fatty acids is then directed into a propane-separation unit 7, and more specifically through a separator 9 and/or a distillation column 11, preferably through both a separator 9 and a distillation column 11. The separator 9 can for example remove water as indicated on figure 1. A stream 13 comprising propane is then generated. An additional effluent 21 of one or more fatty acids can also optionally be recovered from the propane-separation unit 7. Further treatment (not shown) of such one or more fatty acids, can generate naphtha and/or diesel. It is noted that such naphtha and/or diesel can also be called green naphtha and/or green diesel, due to their origin. After exiting the propane-separation unit 7, the stream 13 comprising propane is directed towards at least one dehydrogenation reactor 15 comprising one or more conducting catalytic membranes, so that a first effluent 19 comprising at least propylene is generated. At the same time, hydrogen is produced, and can be recovered as a second effluent 17, which can be preferably used at least partially, or in full, in the hydrogenation reaction performed into the hydrotreating unit 5 onto the feed 1. It is also noted that the hydrogen recovered as a second effluent 17 can be called green hydrogen.

For example, the one or more proton-conducting catalytic membranes 15 can be selected from a proton-conducting catalytic membrane comprising an anode, an electrolyte layer disposed on top of the anode and a porous cathode disposed on top of the electrolyte layer, wherein said at least one dehydrogenation catalyst is comprised within the anode; a palladium membrane; a palladium-silver alloy membrane; a silica/alumina membrane; a zeolite-based membrane; a ceramic membrane; a ceramic electrolytic membrane; or a polymeric membrane. Any kind of proton-conducting catalytic membranes known in the art is suitable to be integrated in the propane dehydrogenation process of the present disclosure.

In particular, the present disclosure relates to a propane dehydrogenation (PDH) process remarkable in that it comprises the following steps:

a) providing a feed 1 comprising one or more vegetable oils;

b) providing an hydrogen stream 3 and performing an hydrogenation reaction on said feed 1 to generate at least one effluent 6 comprising propane and one or more fatty acids;

c) separating propane from said one or more fatty acids comprised within said at least effluent 6 generated at step (b) to generate a stream 13 comprising propane;

d) providing at least one proton-conducting catalytic membrane, each proton-conducting catalytic membrane comprising an anode, an electrolyte layer disposed on top of the anode and a porous cathode disposed on top of the electrolyte layer, wherein said at least one dehydrogenation catalyst is comprised within the anode;

e) feeding within the anode of said one or more proton-conducting catalytic membranes under propane dehydrogenation conditions the stream 13 generated at step (c);

f) recovering a first effluent 19 comprising at least propylene.

By using a proton-conducting catalytic membrane integrating one or more dehydrogenation catalysts within an anode acting as support layer of said proton-conducting catalytic membrane itself, this results in a favourable extraction of the hydrogen by expanding the surface contact between the one or more dehydrogenation catalysts and the membrane. *In*

*fine,* this has the beneficial effect of displacing the chemical equilibrium towards the products during a dehydrogenation reaction according to Le Chatelier's principle and subsequently enhancing the conduct of the PDH process by providing among other a conversion superior to 40%, preferably superior to 50%.

More particularly, the present disclosure relates to a propane dehydrogenation (PDH) process remarkable in that it comprises the following steps:

a) providing a feed 1 comprising one or more vegetable oils;
b) providing an hydrogen stream 3 and performing an hydrogenation reaction on said feed 1 to generate at least one effluent 6 comprising propane and one or more fatty acids;
c) separating propane from said one or more fatty acids comprised within said at least one effluent 6 generated at step (b) to generate a stream 13 comprising propane;
d) providing at least one proton-conducting catalytic membrane, each proton-conducting catalytic membrane comprising an anode, an electrolyte layer disposed on top of the anode and a porous cathode disposed on top of the electrolyte layer; wherein the anode comprises one or more channels, each of said channels comprising at least one dehydrogenation catalyst; wherein said one or more channels are flared depth channels or rectangular channels, and wherein said one or more channels are made in copper or steel;
e) feeding within the anode of said one or more proton-conducting catalytic membranes under propane dehydrogenation conditions the stream 13 generated at step (c);
f) recovering a first effluent 19 comprising at least propylene.

Even more particularly, the present disclosure relates to a propane dehydrogenation (PDH) process remarkable in that it comprises the following steps:

a) providing a feed 1 comprising one or more vegetable oils;
b) providing an hydrogen stream 3 and performing an hydrogenation reaction on said feed 1 to generate at least one effluent 6 comprising propane and one or more fatty acids;
c) separating propane from said one or more fatty acids comprised within said at least one effluent 6 generated at step (b) to generate a stream 13 comprising propane;
d) providing at least one proton-conducting catalytic membrane, each proton-conducting catalytic membrane comprising an anode, an electrolyte layer disposed on top of the anode and a porous cathode disposed on top of the electrolyte layer; wherein the anode comprises one or more channels, each of said channels comprising at least one dehydrogenation catalyst; wherein said one or more channels are flared depth channels, and wherein said one or more channels are made in copper or steel;
e) feeding within the anode of said one or more proton-conducting catalytic membranes under propane dehydrogenation conditions the stream 13 generated at step (c);
f) recovering a first effluent 19 comprising at least propylene.

For example, the present disclosure relates to a propane dehydrogenation (PDH) process remarkable in that it comprises the following steps:

a) providing a feed 1 comprising one or more vegetable oils;
b) providing an hydrogen stream 3 and performing an hydrogenation reaction on said feed 1 to generate at least one effluent 6 comprising propane and one or more fatty acids;
c) separating propane from said one or more fatty acids comprised within said at least one effluent 6 generated at step (b) to generate a stream 13 comprising propane;
d) providing at least one proton-conducting catalytic membrane, each proton-conducting catalytic membrane comprising an anode, an electrolyte layer disposed on top of the anode and a porous cathode disposed on top of the electrolyte layer; wherein the anode comprises one or more channels, each of said channels comprising at least one dehydrogenation catalyst; wherein said one or more channels are flared depth channels or rectangular channels, and whrein said one or more channels are made in copper or steel;
e) feeding within the anode of said one or more proton-conducting catalytic membranes under propane dehydrogenation conditions the stream 13 generated at step (c);
f) recovering a first effluent 19 comprising at least propylene;

wherein the anode is devoid of pores, and wherein the electrolyte layer comprises one or more perovskite materials with one or more cations selected from Ba, Ce, Zr, Y, La, Sr, Mn, Fe, Eu, Tb, Ni, Yb, Pr, Sm, Zn, Gd, Er, Co, Nd, or any mixtures thereof.

For example, each triglyceride has three aliphatic chains, and the process is remarkable in that at least two of the

three aliphatic chains are identical and have a number of carbons ranging between 7 and 22, or between 10 and 20, or between 16 and 20, or between 16 and 18. With preference, each triglyceride has three aliphatic chains, and the process is remarkable in that the three aliphatic chains are identical and have a number of carbons ranging between 7 and 22; or between 10 and 20, or between 16 and 20, or between 16 and 18.

The hydrogenation reaction performed in step (b) can be carried out at a flow of at most 200 T/hour, or at most 190 T/hour, or at most 180 T/hour, or at most 170 T/hour, or at most 160 T/hour, or at most 150 T/hour, or at most 140 T/hour, or at most 130 T/hour, or at most 120 T/hour, or at most 110 T/hour, or at most 100 T/hour, or of at most 90 T/hour, or of at most 80 T/hour.

Advantageouslly, the hydrogenation reaction of the one or more vegetable oils and/or of the one or more triglycerides is carried out during a hydrotreating processs of said one or more vegetable oils and/or of said one or more triglycerides. For example, said hydrogenation reaction occurs simultaneously with hydrodenitrogenation (HDN), hydrodesulfurization (HDS), hydrodearomatization (HDA), hydrodemetallization (HDM), and/or partial thermal cracking.

With preference, a step of pre-treating said one or more vegetable oils and/or said one or more triglycerides is carried out before the hydrotreating process. For example, the pre-treating of said one or more vegetable oils and/or said one or more triglycerides comprises a step of degumming carried out before a step of bleaching.

Advantageously, step (g) is carried out, and the process is remarkable in that the second effluent 17 recovered can be used, at least partially or in full, in said hydrogenation reaction performed at step (b). For example, the part of the second effluent comprising hydrogen that is recovered when step (g) is carried out and that is used in said hydrogenation reaction can amount to between 3 wt.% and 10 wt.% of the total weight of the hydrogen stream, preferably between 3.1 wt.% and 6.0 wt.%, or between 3.2 w.% and 5.5 wt.%, or between 3.3 wt.% and 5.5 wt.%, or between 3.3 wt.% and 5.3 wt.%, or between 3.4 wt.% and 5.0 wt.%.

Advantageously, step (g) is carried out, and the process is remarkable in that the second effluent 17 recovered can be used, at least partially or in full, during a hydrotreating process of the feed 1 provided at step (a), namely on a feed comprising one or more vegetable oils and/or one or more triglycerides oils, said hydrotreating process preferably comprising said hydrogenation reaction of said feed 1 performed at step (b).

Advantageously, step (g) is carried out, and the process is remarkable in that the second effluent 17 recovered can be used, at least partially or in full, during a hydroizomerization process of one or more hydrotreated oils that can be preferentially generated from a hydrotreating process of the feed 1 provided at step (a); said hydrotreating process comprising preferably said hydrogenation reaction of said feed 1 performed at step (b).

Indeed, after the hydrogenation reaction of the one or more vegetable oils and/or of the one or more triglycerides, one or more hydrogenated oils are generated. As the hydrogenation reaction preferably occurs during a hydrotreating processs of said one or more vegetable oils and/or said one or more triglycerides, after said hydrotreating process, one or more hydrotreated oils are generated. Advantageously, a process of hydroizomerization (HDI) of said one or more hydrotreated oils and/or of said one or more hydrogenated oils can be carried out. The hydroizomerization process can be used to improve the properties of the hydrotreated oils, such as the lowering of the cloud point.

Advantageously, the stream 13 comprising propane generated at step (c) further comprises steam, preferably in an amount ranging between 1 vol.% and 15 vol.% based on the total volume of the steam 13 comprising propane generated at step (c); more preferably ranging between 2 vol.% and 10 vol.%. For example, the amount of steam in the stream 13 comprising propane generated at step (c) is of at least 2.5 vol.% based on the total volume of the stream 13 comprising propane generated at step (c), preferably of at least 5.0 vol.%, more preferably 7.5 vol.%. For example, the process further comprises the step of providing steam into the stream 13 comprising propane generated at step (c). Said step of providing steam is performed continuously or at intervals (i.e., pulsed), preferably at intervals since this allows to remove coke that can be formed.

Advantageously, the proton-conducting catalytic membrane can be a co-ionic catalytic membrane. **Figure 7** displays a representation of the mechanism involving a co-ionic catalytic membrane. When a co-ionic membrane is used, water is co-fed with the feedstream (such as the feedstream of propane). The water is injected through the porous cathode of catalytic membrane, so that the water is split into hydrogen and oxygen. Then the oxygen anions can cross the membrane, so as to react with part of the hydrogen that is formed at the anode side, namely within the anode of the catalytic membrane. Such co-ionic catalytic membrane conducts therefore protons versus electrons and oxygen anions. Typical materials suitable for the co-ionic catalytic membranes are perovskites $ABO_3$, such as oxides of Co, oxides of CoFe, oxides of Zr, oxides of BaZr, oxides of CaZr doped with one or more of Sr, Ba, Co, Cu, Fe, Cr, La, Ni, Ca.

With preference, the control of the quantity of coke that is formed can also occur when water is co-fed with the feedstream (such as the feedstream of propane), and without the co-ionic catalytic membrane, but only in presence of the proton-conducting catalytic membrane.

The one or more dehydrogenation reactors 15 used in the installation of the present disclosure each comprise a proton-conduction catalytic membrane, with an anode, an electrolyte layer disposed on top of the anode and a porous cathode disposed on top of the electrolyte layer, and wherein the anode preferably comprises one or more channels, each of said channels comprising at least one dehydrogenation catalyst, wherein said one or more channels are flared depth

channels or rectangular channels, and wherein said one or more channels are made in copper or steel. The proton-conducting catalytic membrane of the present disclosure acts as an extractive membrane that removes the hydrogen that is produced during a dehydrogenation process. It is schematized in **figures 2** and **4** and imaged by scanning electron microscopy in **figure 3.**

The proton-conducting catalytic membrane is made according to the following method, which comprises the following steps:

a) providing a mixture of one or more electrolytes and one or more second metals to form a solution of mixed oxides;
b) pressing uniaxially the mixture provided at step (a) to form a porous cathode, for example at a force ranging between 30 kN and 50 kN, preferably between 35 kN and 45 kN;
c) optionally, calcining said porous cathode;
d) providing one or more electrolytes;
e) screen-printing said one or more electrolytes on the porous cathode or on the calcined porous cathode if step (c) is carried out, to obtain an electrochemical cell with an electrolyte layer;
f) sintering said electrochemical cell;
g) providing an electroconductive layer made of one or more first metals and/or of one or more spinels;
h) depositing at least one dehydrogenation catalyst on said electroconductive layer provided at step (g), so as to form a catalytic electroconductive layer;
i) assembling together the electrochemical cell of step (f) with the catalytic electroconductive layer formed at step (h), preferably by sealing with one or more sealants, so as to obtain a proton-conducting catalytic membrane in accordance with the present disclosure.

The screen-printing technology, used at step (e), is a printing technique where a mesh, for example a 21 mesh, is used to transfer an ink onto a substrate. In this case, the substrate is a porous layer which corresponds to the cathode and the ink is made essentially of the electrolyte.

The steps (a) to (f) are the steps for preparing a sintered electrochemical cell with an electrolyte layer, which corresponds to the cathode of a proton-conducting membrane or of a proton-conducting catalytic membrane.

Before step (a), an activation step can be carried out under activation conditions on the oxidized form of the one or more second metals. For example, the activation conditions comprise providing a reduction atmosphere comprising preferably between 15 vol.% and 50 vol.% of hydrogen and between 85 vol.% and 50 vol.% of an inert gas based on the total volume of the reduction atmosphere. For example, the inert gas is Ar, He, $N_2$ or a mixture thereof, preferably Ar. For example, the activation conditions comprise a temperature ranging between 600°C and 800°C, preferably between 650°C and 750°C. For example, the activation conditions comprise a reduction time ranging between 5 hours and 15 hours, preferably between 7 hours and 13 hours. With preference, a calcination step is carried out before said activation step, preferably at a temperature ranging between 600°C and 800°C, more preferably between 650°C and 750°C. For example, the calcination step is carried out under an inert gas atmosphere, such as under Ar, He, $N_2$ or a mixture thereof, preferably under Ar.

The one or more electrolytes provided at step (a) can be a solid solution of at least two perovskite materials. The mixture of step (a) can further comprise one or more polar aprotic solvents, such as one or more of acetone, dimethyl formamide, dimethyl sulfoxide, or a mixture thereof, preferably acetone.

The mixture provided at step (a) can further comprise, to favour the pressing, one or more polymers selected from polyvinyl alcohol (PVA) or poly(methyl methacrylate) (PMMA), preferably polyvinyl alcohol (PVA). For example, the mixture provided at step (a) comprises said one or more polymers and mixed oxides at a ratio ranging between 1/0.050 and 1/0.100, preferably between 1/0.060 and 1/0.090, more preferably between 1/0.070 and 1/0.080.

Step (a) can be performed by grinding the mixture of one or more electrolytes and one or more second metals, preferably for at least 12 hours, more preferably for a time ranging between 18 hours and 36 hours, even more preferably ranging between 20 hours and 32 hours.

After step (a) and before step (b), a drying step can be performed. For example, the drying step is performed at a temperature ranging between 40°C and 80°C, preferably between 50°C and 70°C.

The one or more first metals are advantageously selected from Cu, Ag, Ni, Pt, Pd, Au, Mn, or any mixtures thereof, more preferably Cu and/or Ag. The one or more first metals and/or the one or more spinels can be doped with one or more dopants, preferably selected from Cu, Li, Cr or a mixture thereof.

The one or more second metals are advantageously selected from Ni, Fe, Co, Pd, Pt, or any mixtures thereof, more preferably Ni.

When step (c) is carried out, said step (c) can be performed at a temperature ranging between 600°C and 800°C, preferably between 650°C and 750°C; and/or during a time ranging between 5 hours and 15 hours, preferably between 7 hours and 13 hours.

The one or more electrolytes provided at step (a) and/or at step (d) can be or comprise one or more perovskite

materials with one or more cations selected from Ba, Ce, Zr, Y, La, Sr, Mn, Fe, Eu, Tb, Ni, Yb, Pr, Sm, Zn, Gd, Er, Co, Nd, or any mixtures thereof; with preference, said one or more cations are selected from Ba, Ce, Zr, Y, Ni or any mixture thereof. In addition, the use of one or more perovskite materials with one or more cations selected from Ba, Ce, Zr, Y, La, Sr, Mn, Fe, Eu, Tb, Ni, Yb, Pr, Sm, Zn, Gd, Er, Co, Nd, or any mixtures thereof as the electrolyte layer favours the proton transport from the anode to the cathode. For example, they are or comprise one or more perovskite materials with an electrical conductivity ranging between $10^{-4}$ S/cm and $10^{-3}$ S/cm as determined by electrochemical impedance spectroscopy measurements performed at 600°C under an atmosphere comprising Ar and $H_2$ in a ratio 75/25. Advantageously, the one or more electrolytes provided at step (d) are the same as the one or more electrolytes provided in the mixture with one or more second metals.

Step (f) can be performed at a temperature ranging between 1300°C and 1700°C, preferably between 1350°C and 1650°C, more preferably between 1400°C and 1600°C

Step (f) can be performed for a time of at least 5 hours, preferably between 10 hours and 15 hours.

The steps (g) and (i) are the steps of making an anode on the electrolyte layer of the sintered electrochemical cell, the anode comprising ideally one or more dehydrogenation catalysts provided at step (h) so that the proton-conducting membrane is a proton-conducting catalytic membrane and can be used for example in a dehydrogenation process.

Before step (h) a step of forming one or more channels within the electroconductive layer provided at step (g) can be carried out. With preference, said step of forming one or more channels is performed by electroforming or electroplating the electroconductive layer provided at step (g). For example, said step of forming one or more channels is performed by chemical vapour deposition (CVD), physical vapour deposition (PVD), thermal spraying, microfabrication by etching, photolithography, tri-dimensional printing (such as fused deposition modelling, stereolithography or selective laser sintering), machining (such as milling, drilling or stamping), or any combination thereof.

Before step (i), a step of covering the electrochemical cell of step (f) with a layer of the same one or more first metals as those making the electroconductive layer provided at step (g) can be carried out. The step (i) is advantageously performed by sealing together the electrochemical cell of step (f) with the catalytic electroconductive layer formed at step (h) with one or more sealants. For example, the one or more sealants are one or more sealing tapes and/or one or more sealing pastes, more preferably one or more sealing tapes.

<u>The anode</u>

The anode is an electroconductive layer. The anode comprises at least one dehydrogenation catalyst. The anode is preferably devoid of pores.

For example, the electroconductive layer is made of one or more first metals and/or of one or more spinels (i.e., $MgAl_2O_4$). This corresponds to an anode, where hydrogen is transformed into protons ($H^+$), following the chemical equation (1):

$$H_2 \rightarrow 2\ H^+ + 2e^-$$

With preference, the one or more first metals are selected from Cu, Fe, Cr, Ag, Ni, Mo, Pt, Pd, Au, Mn or any mixtures thereof, more preferably from Cu, Fe, Cr, Ag, Ni, Mo, Pt, Pd or any mixtures thereof, even more preferably from Cu, Fe, Cr, Ag, Ni, Mo or any mixtures thereof, most preferably from Cu, Fe, Cr, Ag, Ni or any mixtures thereof, such as Cu, Fe, Cr, Ni or any mixtures thereof, or such as Cu, Fe, Cr or any mixtures thereof, or such as Cu and/or Ag. Silver is the highest conducting metal while copper is the cheapest. The one or more first metals and/or the one or more spinels can be preferably doped with one or more dopants which can be selected from Cu, Li, Cr or a mixture thereof.

Advantageously, the anode is made of or comprises steel.

For example, the anode is made of or comprises stainless steel, more preferably ferritic stainless steel.

For example, the steel comprises between 60 wt.% and 80 wt.% of iron based on the total weight of the steel, preferably between 63 wt.% and 75 wt.% of iron.

For example, the steel comprises between 11 wt.% and 18 wt.% of chromium based on the total weight of the steel, preferably between 12 wt.% and 17 wt.%.

For example, the steel comprises between 10 wt.% and 14 wt.% of nickel based on the total weight of the steel, preferably between 11 wt.% and 13 wt.%.

For example, the steel comprises between 1 wt.% and 3 wt.% of molybdenum, such as 2 wt.% of molybdenum.

For example, the steel comprises less than 0.15 wt.% of carbon based on the total weight of the steel, preferably less than 0.12 wt.%.

For example, the steel comprises between 60 wt.% and 80 wt.% of iron, between 11 wt.% and 18 wt.% of chromium and less than 0.15 wt.% of carbon, based on the total weight of the steel.

For example, the steel comprises at least 60 wt.% of iron and between 11 wt.% and 18 wt.% of chromium based on the total weight of the steel. With preference, the steel also comprises less than 0.15 wt.% of carbon based on the total

weight of steel; preferably less than 0.12 wt.%.

In particular, the steel comprises at least 60 wt.% of iron, between 11 wt.% and 18 wt.% of chromium, and less than 0.15 wt.% of carbon, based on the total weight of the steel. More particularly, the steel comprises at least 60 wt.% of iron, between 11 wt.% and 18 wt.% of chromium, less than 0.15 wt.% of carbon, between 10 wt.% and 14 wt.% of nickel and between 1 wt.% and 3 wt.% of molybdenum, based on the total weight of the steel.

Advantageously, the anode has a thickness ranging between 3 mm and 6 mm as designed by SolidWorks software, preferably between 3.5 mm and 5.5 mm. A reactor housing, made for example in Inconel or steel, can encompass the anode, such as an anode made of copper.

When the anode is made of steel, there is no need of reactor housing. Therefore, the thickness of the whole reactor is ranging between 1 cm and 2 cm. As no reactor housing is required, the reactor is lighter compared to a reactor with an anode in copper which requires an Inconel or a steel housing.

For example, the anode is non-porous.

Advantageously, the one or more dehydrogenation catalysts comprise one or more metal-based catalysts, one or more metal oxide-based catalysts, one or more zeolites, one or more transition metal carbides, one or more transition metal nitrides, one or more carbon nanotubes, or any mixtures thereof. With preference, the one or more dehydrogenation catalysts comprise one or more metal-based catalysts, one or more metal oxide-based catalysts, one or more zeolites, or any mixtures thereof. For example, the one or more zeolites are selected from the group of CHA, MFI families or any mixtures thereof. For example, the one or more zeolites are doped with one or metals selected from Mo, W, Fe, V, Cr or any mixtures thereof. For example, the one or more metal-based catalysts and/or the one or more metal oxide-based catalyst comprises one or more metals selected from Pd, Pt, Sn, Mo, W, Fe, V, Cr or any mixtures thereof. The metal-based catalysts, such as the catalysts comprising one or more metals selected from Pd, Pt, Sn, Mo, W, Fe, V, Cr or any mixtures thereof, or preferably selected from Pd, Pt, Sn or any mixture thereof, are preferred as dehydrogenation catalysts. Such metal-based catalysts can be supported, for example onto $Al_2O_3$, SiOz, $TiO_2$, $CeO_2$ or any mixture thereof, more preferably $Al_2O_3$ and/or $SiO_2$.

The fact that the anode comprises the one or more dehydrogenation catalysts allows for maximisation of the contact surface between the one or more dehydrogenation catalysts and the electrolyte, leading therefore to an improvement of the extraction capability of the membrane.

<u>The electrolyte layer</u>

The electrolyte layer acts as a proton transport layer. It is noted that since the electrolyte is a medium containing ion that is electrically conducting upon the movement of those ions, alkanes such as propane, or the products of the dehydrogenation reaction, namely corresponding alkenes, such as propene, cannot cross the electrolyte layer. Only hydrogen under the form of proton (H+) will be able to cross the electrolyte layer and then extracted upon application of an electrical current.

For example, the electrolyte of the electrolyte layer comprises one or more perovskite materials with an electrical conductivity ranging between $10^{-4}$ S/cm and $10^{-3}$ S/cm as determined by electrochemical impedance spectroscopy measurements performed at 600°C under an atmosphere comprising Ar and $H_2$ in a ratio 75/25.

For example, the electrolyte of the electrolyte layer comprises one or more perovskite materials having a general formula $ABX_3$, wherein A and B are cations with different oxidation states and X is an anion. The A-cation occupies the center of the unit cell, while the B cation and the X anions (commonly oxygen) are arranged at the corners and the edges of the unit cell, respectively.

For example, the electrolyte of the electrolyte layer comprises one or more perovskite materials with one or more cations selected from Ba, Ce, Zr, Y, La, Sr, Mn, Fe, Eu, Tb, Ni, Yb, Pr, Sm, Zn, Gd, Er, Co, Nd, or any mixtures thereof. With preference, said one or more cations are selected from Ba, Ce, Zr, Y, Ni or any mixtures thereof. More preferably, the one or more cations are selected from Ba, Ce, Zr, Y, or any mixtures thereof. For example, the electrolyte comprises at least two perovskite materials, preferably in the form of a solid solution. The use of such cations as the electrolyte layer favours the proton transport from the anode to the cathode.

For example, the electrolyte of the electrolyte layer comprises one or more perovskite materials being $BaCeO_3$ and $BaZrO_3$ in the form of a solid solution. $BaCeO_3$ exhibits higher proton conductivity than BaZrOs, but can suffer chemical instability. On the other hand, $BaZrO_3$ presents adequate stability under different conditions but presents a significant grain boundary resistance in addition to the high sintering temperature that causes Ba evaporation and the subsequent loss of transport properties. The solid solution of both $BaCeO_3$ and BaZrOs, corresponding to the electrolyte $BaCe_{0.3}Zr_{0.5}Y_{0.2}O_3$, can overcome the disadvantages of both materials taken independently.

Advantageously, the electrolyte layer has a thickness ranging between 20 μm and 40 μm as determined by scanning electron microscopy, preferably between 25 μm and 35 μm.

With preference, the anode, which is an electroconductive layer, comprises one or more channels arranged in parallel to each other, each of said channels comprising the one or more dehydrogenation catalysts. For example, the

anode can advantageously comprise two channels, preferably 4 channels, more preferably 8 channels, or 9 channels or 10 channels. A configuration in which an anode comprises 4 channels is schematized in **figure 4.** The fact that the one or more dehydrogenation catalysts are surrounded by the electrolyte further contributes to the extractive capabilities of the proton-conducting catalytic membrane of the present disclosure. For example, each of said one or more channels has a cross-section amounting to at least 2 $cm^2$, or to at least 2.5 $cm^2$. For example, at least a part of said one or more channels are rectangular channels (see **figure 5**), flared depth channels (see **figure 6**), flared width channels, bended channels, or corrugated channels; more preferably, all of said one or more channels are rectangular channels, flared depth channels, flared width channels, bended channels, or corrugated channels; even more preferably, all of said one or more channels are rectangular channels or flared depth channels, most preferably, all of said one or more channels are flared depth channels. In particular, flared depth channels are channels with a surface area of the outlet larger than the surface area of the inlet, the inlet and the outlet being defined according to the sense of the feed flow, for example the inlet is where the compound to be dehydrogenated (*e.g.*, the propane) is introduced into the proton-conducting catalytic membrane and the outlet is where the one or more products of the dehydrogenation reaction (*e.g.*, propylene, cracked products (such as ethane and/or methane), coke) are exiting the proton-conducting catalytic membrane.

The porous cathode

For example, the porous cathode comprises a mixture of an electrolyte and one or more second metals. This corresponds to an electrochemical cell with a porous cathode, which ensures the recombination of protons ($H^+$) into hydrogen ($H_2$), following the chemical equation (2):

$$2H^+ + 2e^- \rightarrow H_2$$

With preference, the one or more second metals are selected from Ni, Fe, Co, Pd, Pt, or any mixtures thereof, more preferably Ni. For example, the amount of the one or more second metals is ranging between 50 wt.% and 70 wt.% of the total weight of said mixture, more preferably between 55 wt.% and 65 wt.%. Thus in the case where nickel would be selected as the preferred cathodic metal, the amount of nickel in the mixture forming the porous cathode is ranging between 50 wt.% and 70 wt.% of the total weight of said mixture, more preferably between 55 wt.% and 65 wt.%.

Advantageously, the porous cathode is a porous layer with a thickness ranging between 30 μm and 50 μm as determined by scanning electron microscopy, preferably between 35 μm and 45 μm.

The proton-conducting catalytic membrane

The proton-conducting membrane conducts protons versus electrons.

Advantageously, the proton-conducting catalytic membrane of the present disclosure has a hydrogen extraction ratio ranging between 0.20 and 0.99, or between 0.25 and 0.90 at a temperature of at least 500°C and/or at a pressure of at most 0.1 MPa; the hydrogen extraction ratio being simulated by computational fluid dynamics (CFD) and/or quantified by gas chromatography (GC) during the reaction. With preference, the proton-conducting catalytic membrane presents a hydrogen extraction ratio ranging between 0.20 and 0.99, or between 0.25 and 0.90 at a temperature of at least 550°C and/or at a pressure of at most 0.05 MPa.

For example, the hydrogen extraction ratio can be of at least 0.2 at at least 600°C, the hydrogen extraction ratio being simulated by computational fluid dynamics (CFD), or the hydrogen extraction ratio can be of at least 0.5 at at least 575°C.

Advantageously, the proton-conducting catalytic membrane can be a co-ionic catalytic membrane. **Figure 7** displays a representation of the mechanism involving a co-ionic catalytic membrane. When a co-ionic membrane is used, water is co-fed with the feedstream (such as the feedstream of propane). The water is injected through the porous cathode of catalytic membrane, so that the water is split into hydrogen and oxygen. Then the oxygen anions can cross the membrane, so as to react with part of the hydrogen that is formed at the anode side. Such catalytic membrane conducts therefore protons versus electrons and oxygen anions and can therefore be referred to as a co-ionic catalytic membrane. Typical materials suitable for co-ionic catalytic membranes are perovskites $ABO_3$, such as oxides of Co, oxides of CoFe, oxides of Zr, oxides of BaZr, oxides of CaZr doped with one or more of Sr, Ba, Co, Cu, Fe, Cr, La, Ni, Ca.

With preference, the control of the quantity of coke that is formed can also occur when water is co-fed with the feedstream (such as the feedstream of propane), and without the co-ionic catalytic membrane, but only in presence of the proton-conducting catalytic membrane.

The dehydrogenation reactor

The present disclosure also relates to a dehydrogenation reactor comprising at least one proton-conducting

catalytic membrane. With preference, the dehydrogenation reactor has a planar geometry, reducing subsequently the gas polarization inside the reactor. It is therefore possible to further improve the extracting capability of the proton-conducting catalytic membrane of the present disclosure. Such dehydrogenation reactor further minimizes coking formation and therefore preserves the activity of the one or more dehydrogenation catalysts.

A method for preparing the dehydrogenation reactor is also described. Said method of preparation comprises the following steps:

a) providing a reactor, preferably a reactor with a planar geometry;
b) inserting the proton-conducting catalytic membrane as defined above within said reactor.

For example, when said dehydrogenation reactor comprises more than one proton-conducting catalytic membrane, said dehydrogenation reactor further comprises a spacer between each proton-conducting membrane.

For example, the spacer is an electroconducting plate, preferably a steel plate, more preferably a stainless steel plate, even more preferably a ferritic stainless steel plate. For example, the steel plate is doped with one or more oxides (such as oxides of La and/or Y), one or more metals, (such as metals selected from Au, Ni, Pt, Cu, Pd, Ti, W or a mixture thereof), one or more metallic alloys (such as one or more ferritic alloys and/or one or more chromite alloys) or a mixture thereof. The dehydrogenation reactor can thus be advantageously an arrangement of several proton-conducting catalytic membranes. For example, the arrangement of several proton-conducting catalytic membranes can comprise two or more proton-conducting catalytic membranes, preferably between 4 and 20 proton-conducting catalytic membranes.

The several proton-conducting catalytic membranes can be arranged on top of each other, so as in **figure 8** (namely a stacking of 4 membranes) or in **figure 9** (namely a stacking of 20 membranes), or in an adjacent manner, wherein each proton-conducting catalytic membrane can be arranged next to each other on the same level. In another configuration, the several proton-conducting catalytic membranes can be arranged both on top of each other and in an adjacent manner.

Advantageously, said reactor has an inlet and-an outlet, and said reactor is remarkable in that the inlet is in a first zone arranged so that a feed to said reactor via the inlet contacts the one or more dehydrogenation catalysts of the proton-conducting catalytic membrane before passing to the outlet, the outlet being for example in a second zone that is downstream of the proton-conducting catalytic membrane.

Advantageously, said reactor has an inlet and an outlet, and said reactor is remarkable in that the anode of the one or more proton-conducting catalytic membranes comprises one or more channels, each of said channels comprising the one or more dehydrogenation catalysts, and in that the inlet is in a first zone arranged so that a feed to said reactor via the inlet is conducted through the one or more channels and contacts the one or more dehydrogenation catalysts before passing to the outlet being for example in a second zone that is downstream of the proton-conducting catalytic membrane.

With preference, the inlet has surface area identical to the surface area of the outlet or the inlet has a surface area smaller than the surface area of the outlet. More preferably, the inlet has a surface area smaller than the surface area of the outlet, which is the case when the channels within the anode are flared depth channels.

For example, the surface area of the inlet can represent between 25% and 50% of the surface area of the outlet, preferably between 30% and 45%. In other words, the surface area of the outlet can be at least 2 times larger than the surface area of the inlet, more preferably at least 2.5 times, even more preferably at least 2.75 times, most preferably at least 3 times. This gradient in the geometry, wherein the lowest surface area is at the inlet side of the reactor and the bigger surface area is at the outlet side of the reactor, favours the extraction of hydrogen at the outlet side, in accordance with the circulation of the feed within the dehydrogenation reactor.

With preference, the inlet and outlet of the reactor are arranged on faces of the reactor that are opposed to each other. In other words, the inlet is in a first zone arranged so that a feed to said reactor via the inlet is conducted towards the proton-conducting catalytic membrane and the outlet is in a second zone that is downstream of the proton-conducting catalytic membrane. With preference, the first zone and the second zone each form one face of the reactor and are advantageously opposed to each other. Alternatively, the first zone and the second zone are arranged on a single face of the reactor, as shown by the stacking of **figures 8** and **9.**

With preference, said reactor has an additional inlet and an additional outlet arranged on faces opposed to each other. This additional inlet can be used for feeding a carrier gas, such as $H_2$, Ar, HArMix (i.e., a mixture comprising $H_2$ and Ar with an amount of hydrogen comprised between 3 vol.% and 5 vol.% of the total volume of the mixture), or any mixtures thereof, to the dehydrogenation reactor.

**Test and determination methods**

Scanning electron microscopy (SEM) analysis was carried out by using a field-emission scanning electron microscope using a Zeiss Ultra 55 fitted with a field emission gun using an accelerating voltage of 30.0 kV. All samples before the SEM characterization were covered with a conductive layer (Pt or Au).

Electrochemical Impedance Spectroscopy (EIS) measurements were performed to validate the transport properties of the developed membranes. EIS measurements were measured using a SolarTron instrument and by applying an electrical current through the membrane by means of two silver electrodes EIS allows obtaining the resistance of the electrolyte, while equation (1) allows the determination of the electrical conductivity of the material.

$$\sigma\left(\frac{S}{cm}\right) = \frac{1}{R\ (\Omega)} * \frac{t\ (cm)}{\phi\ (cm^2)} \qquad (1)$$

wherein $\sigma$ is the electrical conductivity, R is the electrical resistance and $\phi$ is the area of electrode and t is the thickness of the electrode.

The hydrogen extraction ratio, which is the quantity of hydrogen extracted on the quantity of hydrogen formed, is obtained by simulations using computational fluid dynamics (CFD) technique and/or is determined by using gas chromatography (GC) technique. The hydrogen extraction ratio is calculated and/or determined on the basis of the reactant conversion, for example the propane conversion and in the case of GC, the quantity of hydrogen formed, or in the case of CFD, the theoretical quantity of hydrogen generated.

With respect to the simulations made by CFD technique, they were performed with COMSOL Multiphysics 5.6 software on SYS-6018R-MTR Super server, with an Intel Xeon CPU E5-2640 v4 processor (clock speed 2.4 GHz, 40 CPUs) and 131 Gb RAM running Windows server edition 2016 (64-bit) as an operating system. For the gas flow, Navier-Stokes equations with the respective correction for the porous catalytic bed have been considered. Transport of species has been modelled using averaged-mixture model with propane, propylene and $H_2$ as species. The density of the gas mixture has been estimated considering a mixture of ideal gases, and the viscosity has been calculated using the Wilke model. The porosity and permeability are two key factors that govern the fluid flow in the porous region and the permeability for a packed bed with randomly distributed spherical particles was calculated using the Carman-Kozeny model. According to the publication of Tong Y. et al (Int. J. of Hydrogen Energy, 2022, 47, 12067-12073), electrochemical hydrogen pumps are devices based upon proton-conducting electrolytes that offer 100% selectivity to hydrogen and allow for easy control of the hydrogen separation rate by simply adjusting the applied direct current. The tortuosity was estimated considering the inverse of the square root of the porosity. The binary gas diffusion coefficient has been calculated with an empirical equation based on the Fuller kinetic gas theory, and it was corrected with the ratio of the porosity to tortuosity. The mesh performed was based on tetrahedral elements, where the element size was calibrated for fluid dynamics. The calculations were carried out using the Parallel Direct Solver (PARDISO) with parameter continuation to assure convergence. The relative tolerance of the method is 0.001.

The selectivity in propylene ($C_3$=) is determined according to formula (2):

$$S_{propylene} = \frac{3\,[C_3H_6]}{[CH_4]+2[C_2H_4]+2[C_2H_6]+3[C_3H_6]+3[C_3H_8]} x100 \qquad (2)$$

wherein the numerator is the carbon adjusted molar amount of propylene and the denominator is the sum of all the carbons adjusted molar amount of all hydrocarbons in the effluent.

Propylene yield was determined by multiplying the propane conversion with the propylene selectivity and divided by 100.

The thickness of the anode has been designed using SolidWorks software, which is a solid modelling computer-aided design (CAD) and computer-aided engineering (CAE) application published by Dassault Systems.

The feed flow of the second stream and/or of the purge stream has been determined using a SolarTron ISA flowmeter.

The ampere meter/power supply instrument and the voltmeter/power supply instrument used for determining respectively the electrical current density and the electrical potential is a SolarTron power supply module.

## Examples

The embodiments of the present disclosure will be better understood by looking at the different examples below.

**Preparation of the electrolyte $BaCe_{0.3}Zr_{0.5}Y_{0.2}O_3$ under a form of a solid solution of $BaCeO_3$ and $BaZrO_3$.**

1) The precursors were ground separately for 60 hours. The employed precursors were: BaCOs (99.8% 1-micron powder. Alfa Aesar Ref: 14341, 1kg, CAS: 513-77-9), YSZ ($ZrO_2/Y_2O_3$) (8%),
$CeO_2$ (REacton, 99.9% (REO), 5-micron powder. Alfa Aesar Ref: 11328, 1kg, CAS: 1306-38-3) and $Y_2O_3$ (99.9995%.

ABCR Ref: AB102097, 100 g, CAS:1314-36-9).

2) The different precursors were mixed in adequate proportions in acetone to fulfil the stoichiometry of the desired compound and were ground for 24 hours. The following amount of precursors were weighted:

BaCOs ---> 17.06 g
YSZ → 6.14 g
$CeO_2$ → 4.46 g
$Y_2O_3$ → 1.09 g

3) The compounds were dried by heating at 60 °C in a furnace.
4) The oxide mixture was sieved with a particle size of 200 μm.
5) The oxide mixture was uniaxially pressed at 40 kN in the shape of a disc (disc diameter: 30 mm).
6) Discs were sintered at 1100°C for 10 hours.
7) Steps 4-6 were repeated twice then, the mixture was ground for 24 hours.
8) The calcined oxide mixture was pressed on 20 mm discs at 30kN.
9) The obtained discs were sintered at 1565°C for 12 hours.

**Preparation of the cathode NiO-$BaCe_{0.3}Zr_{0.5}Y_{0.2}O_3$ under a form of a solid solution of $BaCeO_3$ and $BaZrO_3$**

25 g of the solid solution of $BaCeO_3$ and $BaZrO_3$ was then mixed and ground for 15 hours in acetone together with 37.5 g NiO (99% - metal basis; 325 mesh powder, Alfa Aesar Ref: 12359, 250 g, CAS 1313-99-1). After a step of drying by heating at 60°C in a furnace, the mixed oxides were mixed with polyvinyl alcohol (PVA) to favour the pressing. The mixture has a ratio of mixed oxides/PVA 1/0.075. It was then ground with agate mortar before being pressed uniaxially at 40 kN in the shape of a disc (disc diameter: 20 mm). Optionally, the cathode was calcined at 700°C for 10h.

Then, the electrolytes were coated on the cathode by using screen-printing technique using a 21-mesh to obtain a porous cathode with an electrolyte layer. The support is a pressed powder transformed into a disk. In membranes, the screen-printing procedure consists of squeezing the slurry "ink" (electrolyte) to pass through a 21-mesh screen to print it on the cathode. After drying the layer (temperature of 80°C), it is possible to deposit extra layers. The typical thickness of the obtained layer is around 30 μm. After that, the different layers and cathode were sintered at 1565 °C to obtain an efficient attachment between the cathode and the electrolyte and to afford a sintered porous cathode with an electrolyte layer.

Prior to be used, the NiO species have been reduced under hydrogen to form $Ni^+$. This activation step is carried out in a 25 vol.% $H_2$ atmosphere in argon at 700°C for 10 hours, optionally with a pre-calcination at 700°C under pure argon for 10 hours before the activation step.

**Preparation of the proton-conducting membrane**

Then, an anodic layer was coated on the electrolyte layer of the sintered porous cathode.

The anodic layer is an anode comprising Ag. A silver conducting paint (DYNALOY® 342) was indeed obtained from Merck (CAS 7440-22-4) and painted by hand.

**Figure 10** shows a representative example of the electrochemical impedance spectra used to calculate the electrical conductivity. Three batches proton-conducting membrane, namely of electrochemical cells ($BaCe_{0.3}Zr_{0.5}Y_{0.2}O_3$ on uncalcined NiO-$BaCe_{0.3}Zr_{0.5}Y_{0.2}O_3$) layered with an Ag anode have been used. The curve "1" corresponds to wet conditions test (*i.e.*, 3 vol.% of water in the $H_2$/Ar feed) while the curves "2" and "3" are under the same dry conditions (*i.e.*, $H_2$/Ar feed without water) (reproducibility test). The tests have been made at 700°C. The intersect with the abscissa (that can be viewed on the zoom of **figure 10** provided at **figure 11)** gives a value used for plotting **figure 12.**

**Figure 12** shows the conductivity of three batches of proton-conducting membranes, namely of electrochemical cells (**1A**: $BaCe_{0.3}Zr_{0.5}Y_{0.2}O_3$ on uncalcined NiO-$BaCe_{0.3}Zr_{0.5}Y_{0.2}O_3$ with an activation preceded by a calcination; **1B:** $BaCe_{0.3}Zr_{0.5}Y_{0.2}O_3$ on uncalcined NiO-$BaCe_{0.3}Zr_{0.5}Y_{0.2}O_3$ with an activation without calcination; and **2A:** ($BaCe_{0.3}Zr_{0.5}Y_{0.2}O_3$ on calcined NiO-$BaCe_{0.3}Zr_{0.5}Y_{0.2}O_3$) layered with an Ag anode.

**1A:** NiO-$BaCe_{0.3}Zr_{0.5}Y_{0.2}O_3$ is pre-calcinated at 700°C under pure argon for 10 hours before NiO being activated with a 25 vol.% $H_2$ atmosphere in argon at 700°C for 10 hours.

**1B:** NiO is activated with a 25 vol.% $H_2$ atmosphere in argon at 700°C for 10 hours, without a pre-calcination step.

**2A:** NiO-$BaCe_{0.3}Zr_{0.5}Y_{0.2}O_3$ is pre-calcinated at 700°C under pure argon for 10 hours before addition of $BaCe_{0.3}Zr_{0.5}Y_{0.2}O_3$. Then the NiO is reduced under $H_2$ at 700°C for 10 hours.

**Preparation of the proton-conducting catalytic membrane**

The preparation of the reactor assembly was made as following:

25 g of the solid solution of $BaCeO_3$ and $BaZrO_3$ was then mixed and ground for 15 hours in acetone together with 37.5 g of NiO (99% - metal basis; 325 mesh powder, Alfa Aesar Ref: 12359, 250 g, CAS 1313-99-1). After a step of drying by heating at 60°C in a furnace, the mixed oxides were mixed with polyvinyl alcohol (PVA) to favour the pressing. The mixture has a ratio of mixed oxides/PVA 1/0.075. It was then ground with agate mortar before being pressed uniaxially at 40 kN in the shape of a disc (disc diameter: 20 mm). Optionally, the cathode is calcined at 700°C for 10h.

Then, the electrolytes were coated on the cathode by using screen-printing technique using a 21-mesh to obtain a porous cathode with an electrolyte layer. The support is a pressed powder transformed into a disk. In membranes, the screen-printing procedure consists of squeezing the slurry "ink" (electrolyte) to pass through a 21-mesh screen to print it on the cathode. After drying the layer (temperature of 80°C), it is possible to deposit extra layers. The typical thickness of the obtained layer is around 30 μm. After that, the different layers and cathode were sintered at 1565 °C to obtain an efficient attachment between the cathode and the electrolyte and to afford a sintered porous cathode with an electrolyte layer.

Prior to be used, the NiO species have been reduced under hydrogen to form $Ni^+$. This activation step is carried out in a 25 vol.% $H_2$ atmosphere in argon at 700°C for 10 hours, optionally with a pre-calcination at 700°C under pure argon for 10 hours before the activation step.

Then, an anodic layer was coated on the electrolyte layer of the sintered porous cathode.

The anodic layer is an anode comprising Cu, with channels and a dehydrogenation catalyst comprised within said channels.

The sintered porous cathode with an electrolyte layer was covered with copper, using ion-sputtering. The ion-sputtering conditions involved a Pfeiffer Classic 250 deposition system equipped with two RF (13.56 MHz) power sources, each capable of delivering up to 25 W of power. The system utilizes a Cu target for the deposition process and operates at room temperature. The deposition was carried out under a pure Ar atmosphere with a pressure range of $2.6.10^{-2}$ to $7.4.10^{-2}$ mbar.

A channeled support in copper comprising a dehydrogenation catalyst was then prepared by 3D printing of the anode. The 3D printer is a Markeforged MetalX printer using a Bound Powder Filament made of copper (90%) and a polymer (10%). The 3D printing allows to make linear channels. The obtained support was then calcined to remove the polymer at about 200°C and was sintered under Ar at about 900°C for 72h. Then, the channels of the support were filled by hands with 1.25 g of catalyst powder, namely of PtSnEu support on $Al_2O_3$ (0.5 wt.% of Pt, 2 wt.% of Eu and 3 wt.% of Sn).

The sintered porous cathode with an electrolyte layer covered with copper was thus placed on top of the channeled anode comprising the dehydrogenation catalyst and sealed with either GL1734 glass sealing paste (commercially available at Mo-Sci Corporation) or GM31107 glass sealant tape (commercially available at Schott AG) to obtain a proton-conducting catalytic membrane. The glass sealing paste was activated at 620°C for 4 hours, while the glass sealant tape was activated at 700°C for 2 hours.

The proton-conducting catalytic membrane was then placed into a reactor housing in Inconel alloy.

## Hydrogen extraction with the proton-conducting catalytic membrane

The present disclosure introduces a reactor comprising an electrochemical cell based on protonic-conducting materials to extract the generated $H_2$ and shift the equilibrium to the propane dehydrogenation reaction.

The graph displayed in **figure 13** shows the evolution of the electrical potential in function of the area specific resistance (ASR). The best operating cell potential is about 0.5 V and about 0.6 V, although it could be of 1.0 V or even more. However, the maximum value not to cross is 1.8 V, since above this limit, the proton-conducting catalytic membrane will be damaged.

The elemental model represented in **figure 14** (in function of the temperature) and in **figure 15** (in function of the pressure) shows that high current density, and therefore $H_2$ extraction, is required to achieve high propane conversions: at 550°C, the propane equilibrium conversion reaches around 30% for a conventional reactor where no extraction is occurring. The $H_2$ extraction led to clear conversion improvement, notably an extraction of 80 % of the $H_2$ generated is required to achieve conversions higher than 50%.

The $H_2$ extraction is modelled by considering the Faraday Law:

$$F_{H_2\ extracted} = \frac{i}{z.F} . M_{H_2} \qquad (3)$$

wherein the $F_{H_2 extracted}$ is the mass flux of the $H_2$ extracted for the $H_2$ extraction boundary, the $i$ is the current density, the z is the exchanged electrons (two for the protonic exchange), $F$ is the Faraday constant (96485 C/mol) and $M_{H_2}$ is the $H_2$ molecular weight. Other lateral walls were assigned a no-slip boundary condition.

CFD simulations have been performed to study the different parameters affecting the PDH reaction and to optimize the reaction performance. To maximize the membrane surface area to catalyst volume ratio and improve the fluid dynamic regime, micro-channels have been placed in the reaction chamber, namely within the anode of the proton-conducting catalytic membrane of the present disclosure. Those channels are either rectangular or flared depth.

**One or more rectangular channels in the anode**

The initial geometry considered in this study consists of a series of rectangular microchannels arranged in parallel, with a length of 50 mm and a surface area of the inlet being equal to the surface area of the outlet (*e.g.,* 25 $mm^2$), as shown by **figure 5.** These channels have been filled with 1.25 g of catalyst (*i.e.,* PtSnEu supported on $Al_2O_3$) and equipped with a selective membrane to remove $H_2$ on the top surface.

Table 1 shows that the $H_2$ is evacuated from the membrane, subsequently leading to an increase of propane conversion by comparison with a dehydrogenation reactor devoid of the proton-conducting catalytic membrane of the disclosure. The results of table 1 have been simulated by CFD at 550°C and at 0.1 MPa.

Table 1: Simulation by CFD of the propane conversion, using a proton-conducting catalytic membrane with one rectangular channel within the anode, as shown on **figure 5.**

| | Feed flow F (Nml/min) | Space Velocity SV (Nml/h/g) | Maximum current density j ($A/cm^2$) | Propane conversion (%) |
|---|---|---|---|---|
| #1 | 17.4 | 750 | 0.45 | 52 |
| #2 | 17.4 | 300 | 0.54 | 60 |
| #3 | 8.7 | 750 | 0.25 | 55 |
| #4 | 8.7 | 150 | 0.33 | 72 |

The maximum current density can be seen as the maximum hydrogen formed. As less propane is sent to the catalyst in experiment #3 compared to experiment #1, less hydrogen is formed and then less current is needed to extract this hydrogen. However, at similar conditions (same feed flow and same space velocity), a lower current density (as in experiment #1 versus experiment #2, as in experiment #3 versus experiment #4) does not allow a maximum extraction, meaning that the conversion is decreasing.

A lower feed flow allows generally to increase the propane conversion, since the catalyst has time to adsorb and activate more propane.

**One or more flared depth channels in the anode**

However, an optimized geometry study showed that the optimal extraction is performed having a flared depth channel as shown in **figure 6,** namely with a surface area of the outlet (*e.g.,* 25 $mm^2$) being larger than the surface area of the inlet (*e.g.,*10 $mm^2$). In this experiment, the length of the channel has been fixed to 50 mm. These channels have been filled with 1.25 g of catalyst (*i.e.,* PtSnEu supported on $Al_2O_3$)

Table 2 summarizes the results using a dehydrogenation reactor having an outlet with a surface area of 25 $mm^2$ and an inlet with a surface area of 10 $mm^2$. These results have been simulated at a pressure of 0.1 MPa and with a feed flow F of 8.7 Nml/min.

Table 2: Simulation by CFD of the propane conversion using a proton-conducting catalytic membrane with one flared depth channel within the anode, as shown on **figure 6.**

| | Space Velocity SV (Nml/h/g) | Temperature (°C) | Maximum current density ($A/cm^2$) | Propane conversion (%) | Propylene selectivity (%) |
|---|---|---|---|---|---|
| #5 | 750 | 450 | 0.13 | 29 | 99 |
| #6 | 750 | 500 | 0.19 | 44 | 98 |
| #7 | 750 | 550 | 0.25 | 57 | 96 |
| #8 | 750 | 600 | 0.28 | 57 | 91 |
| #9 | 750 | 650 | 0.28 | 73 | 80 |
| #10 | 150 | 450 | 0.17 | 40 | 98 . |
| #11 | 150 | 500 | 0.24 | 58 | 96 |
| #12 | 150 | 550 | 0.30 | 76 | 91 |

(continued)

| | Space Velocity SV (Nml/h/g) | Temperature (°C) | Maximum current density (A/cm$^2$) | Propane conversion (%) | Propylene selectivity (%) |
|---|---|---|---|---|---|
| #13 | 150 | 600 | 0.33 | 87 | 84 |
| #14 | 150 | 650 | 0.32 | 94 | 73 |

It has thus been demonstrated that lower temperatures lead to lower propane conversion and higher propylene selectivity.

It has also been demonstrated that lower space velocity leads to higher propane conversion but lower propylene selectivity and thus more cracking into ethane and methane.

**Improvement over configuration devoid of $H_2$ extraction**

Table 3 indicates the improvement of using a flared depth configuration, in comparison of a proton-conducting catalytic membrane using a rectangular channel configuration or without any proton-conducting catalytic membrane. The simulation has been made at a space velocity SV of 750 Nml/h/g.

Table 3: Simulation by CFD of the propane conversion and the hydrogen mole fraction at the oulet of the proton-conducting catalytic membrane.

| | | Feed flow F (Nml/min) | current density (A/cm$^2$) | Propane conversion (%) | Average $H_2$ mole fraction at the oultet |
|---|---|---|---|---|---|
| #15 | no extraction | 8.7 | 0 | 28.45 | 0.176 |
| | | 17.4 | 0 | 28.70 | 0.180 |
| #16 | $H_2$ extraction using rectangular channels | 8.7 | 0.25 | 48.80 | 0.052 |
| | | 17.4 | 0.25 | 47.18 | 0.066 |
| #17 | $H_2$ extraction using flared depth channels | 8.7 | 0.25 | 54.57 | 0.094 |
| | | 17.4 | 0.25 | 51.51 | 0.051 |

It can be highlighted here that on the outlet side of the dehydrogenation reactor, the conversion of propane is reaching at least 50% when flared depth channels are present within the anode, while for a similar reactor devoid of the proton-conducting catalytic membrane of the disclosure, the conversion of propane is inferior to 30%.

**Dehydrogenation conditions**

Different dehydrogenating conditions including current intensities, feed flows and/or space velocities have been simulated as indicated in table 4 (at constant temperature) and in table 5 (at constant feed flow).

It is noted that when the dehydrogenating step is carried out at 550°C, acceptable side reactions occurred (>95% selectivity).

Table 4: Dehydrogenating step carried out at 550°C.

| | Optimum current intensities | Feed flows | Space velocities | Propane conversion |
|---|---|---|---|---|
| #18 | 0.25 A/cm$^2$ | 8.7 Nml/min | 750 Nml/h/g | 55% |
| #19 | 0.33 A/cm$^2$ | 8.7 Nml/min | 150 Nml/h/g | 72% |
| #20 | 0.45 A/cm$^2$ | 17.4 Nml/min | 750 Nml/h/g | 52% |
| #21 | 0.54 A/cm$^2$ | 17.4 Nml/min | 300 Nml/h/g | 60% |

The highest quantity of formed $H_2$ anticipated by CFD simulations has been obtained without side reactions, at a

feed flow of 17.4 NmL/min; space velocity of 300 NmL/H/g and current intensity of 0.54 A/$cm^2$ (see experiment 21). In such conditions, 10.44 Nml of formed $H_2$, which is below the extraction potential of the membrane for a single channel of 2.5 $cm^2$, were prepared as an example: $H_2$ extraction of 5 $NmL \cdot min^{-1} \cdot cm^{-2}$ meaning 12.5 NmL/min. It showed that the extraction level reached by the disclosure is satisfying. Indeed, through perfect gas law, as it is normal flow rate, then the normal volume is 1 mmol = 22.4 ml. Given that the feed flow is 17.4 Nml/min, the number of moles is amounting to 0.78 mmol. As the conversion is 60% (see experiment 21), the volume of formed $H_2$ is 10.44 Nml.

Under the propane dehydrogenation conditions, the propane can be oxidized into propylene but also other compounds are formed, such as ethylene, ethane and methane. The selectivities were assessed in accordance with the conditions given in table 5.

Table 5: Dehydrogenating step carried out at a feed flow of 8.7 Nml/min

| | Temperature | Optimum current intensities | Space velocities | Propane conversion | Propylene selectivity | Propylene yield |
|---|---|---|---|---|---|---|
| #22 | 450°C | 0.13 A/$cm^2$ | 750 Nml/h/g | 29% | 99% | 28.7% |
| #23 | 450°C | 0.17 A/$cm^2$ | 150 Nml/h/g | 40% | 98% | 39.2% |
| #24 | 500°C | 0.19 A/$cm^2$ | 750 Nml/h/g | 44% | 98% | 43.1% |
| #25 | 550°C | 0.24 A/$cm^2$ | 150 Nml/h/g | 58% | 96% | 55.7% |
| #26 | 550°C | 0.25 A/$cm^2$ | 750 Nml/h/g | 57% | 96% | 54.7% |
| #27 | 550°C | 0.30 A/$cm^2$ | 150 Nml/h/g | 76% | 91% | 69.2% |
| #28 | 600°C | 0.28 A/$cm^2$ | 750 Nml/h/g | 57% | 91% | 51.9% |
| #29 | 600°C | 0.33 A/$cm^2$ | 150 Nml/h/g | 87% | 84% | 73.1% |
| #30 | 650°C | 0.28 A/$cm^2$ | 750 Nml/h/g | 73% | 80% | 58.4% |
| #31 | 650°C | 0.32 A/$cm^2$ | 150 Nml/h/g | 94% | 73% | 68.6% |

Experiment #29 provides a propylene yield of 73.1%.

Furthermore, the CFD simulation results have been plotted as propane conversion and propylene selectivity against the $H_2$ extraction ratio, for different temperatures and space velocities (SV). The flowrate has been set to 8.7 Nml/min, while the current density varied from 0 (no $H_2$ extraction) to a maximum value, which is indicated in table 5. This maximum value corresponds to the point where the $H_2$ molar fraction near the outlet becomes "negative". **Figure 16** shows the results for a space velocity of 150 Nml/h/g and **figure 17** shows the results for a space velocity of 750 Nml/h/g.

As expected, $H_2$ extraction has a clear impact on conversion at relatively low temperatures. Indeed, below 600 °C, the propane conversion could increase by 20-40 % depending on the conditions. On the contrary, at higher temperatures (650 °C), the $H_2$ extraction has little impact on the conversion since the system becomes kinetically limited. However, a higher temperature leads to larger conversion values, but selectivity to propylene decreases. Comparing the space velocities, for SV=150 (i.e., higher catalyst loading), higher conversion values are obtained and $H_2$ extraction affects the conversion a little bit more since kinetic limitations are overcome. But increasing the catalyst loading has an important disadvantage: the activation of the side reactions leads to a lower propylene selectivity.

At SV of 150, an optimum was found between propylene selectivity and propane conversion at 550 °C, propylene selectivity could be over 90 % and propane conversion over 50 % if over 90 % of the $H_2$ produced is extracted (see #32 in table 6).

Table 6: Dehydrogenating step carried out in optimum conditions

| | Temperature | Current intensities | Feed flow | Space velocities | Propane conversion | Propylene selectivity |
|---|---|---|---|---|---|---|
| #32 | 550°C | 0.30 A/$cm^2$ | 8.7 Nml/min | 150 Nml/h/g | 76% | 91% |

Experiment #32 provides a propylene yield of 69.2%.

**Preventing coke formation at temperature superior to 550°C**

**Figure 18** shows that the formation of coke from propylene is occurring at temperature above 550°C, reducing

thus the selectivity into propylene. The formation of coke tends to increase at higher temperature, for example up to 7% of coke at a temperature of 650°C and at an $H_2$ extraction of 96%. The coking generates a loss of selectivity.

The coke deposition and further suppression were modeled by CFD simulations. The two solutions envisioned were water co-feeding and/or water co-feeding with use of a co-ionic membrane, as shown in table 7.

Table 7: Suppression of coke deposition by water co-feeding or water co-feeding along with use of a co-ionic membrane. Experiments were carried out at 575°C, with a current intensity of 0.1 A/$cm^2$ and a time on stream (TOS) of 100 h.

| | | No steam | Water-co-feeding | | Addition of co-ionic membrane | |
|---|---|---|---|---|---|---|
| | Cross-section (cm) | Coke I deposition ($mg_{Coke}/g_{cat}$) | $H_2O$ molar fraction (%) | Coke suppression (%) | $H_2O$ molar fraction (%) | Coke suppression (%) |
| #33 | 0.10 | 0.37 | 2.76 | 100 | 0.1 | 20.5 |
| #34 | 1.25 | 1.14 | 2.28 | 98.3 | 0.6 | 75.9 |
| #35 | 2.50 | 1.27 | 2.07 | 98.1 | 1.2 | 92.8 |
| #36 | 3.75 | 1.32 | 1.95 | 98.5 | 1.7 | 97.3 |
| #37 | 4.90 | 1.35 | 1.86 | 98.8 | 2.1 | 98.7 |

**Figure 19** shows a flared depth channels in which the line A was modelled into graphs showing the evolution of the amount of coke within the channels. Thus, **figure 20** shows the evolution of the coke deposition in function of the reactor length in the absence of steam, while **figure 21** shows the evolution of the coke suppression in function of the reactor length when water is co-fed with the propane and when the reactor employs a proton-conducting catalytic membrane in accordance with the disclosure and **figure 22** shows the evolution of the coke suppression in function of the reactor length when water is co-fed with the propane and when the proton-conducting catalytic membrane of the reactor is a co-ionic membrane.

Both solutions showed coke efficient suppression leading to very stable propylene yield for 100 hours on stream. In contrast, the absence of steam leads to a significant drop in yield after 25 hours on stream.

**Figures 23** to **25** show the evolution of the propylene yield in function of the time on stream (TOS).

**Figure 23** shows that the co-feeding of water into the stream comprising propane at a molar fraction of 2.7% allows to maintain the propylene yield as about 40% at a current density of 0.1 A/$cm^2$.

**Figure 24** shows that the co-feeding of water into the stream comprising propane at a molar fraction of 5.4% allows to maintain the propylene yield as about 50% at a current density of 0.2 A/$cm^2$.

**Figure 25** shows that the co-feeding of water into the stream comprising propane at a molar fraction of 7.9% allows to maintain the propylene yield as about 55% at a current density of 0.3 A/$cm^2$.

**Figure 26** is a view of the reactor assembly in which the anode is made of copper. An external steel housing is shown. The copper anode is for example incorporated within the external steel housing. The scheme shows the channels into the copper anode, that have been made using 3D printing. For example, the reactor assembly has a diameter of 17 cm and a height of 10 cm. The electrical connection to the copper anode must be made through the external steel housing.

**Proton-conducting catalytic membrane with an anode in steel**

The preparation of the reactor assembly (see **figure 27**) was made as following:
25 g of the solid solution of $BaCeO_3$ and $BaZrO_3$ was mixed and ground for 15 hours in acetone together with 37.5 g of NiO (99% - metal basis; 325 mesh powder, Alfa Aesar Ref: 12359, 250 g, CAS 1313-99-1). After a step of drying by heating at 60°C in a furnace, the mixed oxides were mixed with polyvinyl alcohol (PVA) to favour the pressing. The mixture has a ratio of mixed oxides/PVA 1/0.075. It was then ground with agate mortar before being pressed uniaxially at 40 kN in the shape of a disc (disc diameter: 20 mm). Optionally, the cathode is calcined at 700°C for 10 hours.

Then, the electrolytes were coated on the cathode by using screen-printing technique using a 21-mesh to obtain a porous cathode with an electrolyte layer. The support is a pressed powder transformed into a disk. In membranes, the screen-printing procedure consists of squeezing the slurry "ink" (electrolyte) to pass through a 21-mesh screen to print it on the cathode. After drying the layer (temperature of 80°C), it is possible to deposit extra layers. The typical thickness of the obtained layer is around 30 $\mu$m. After that, the different layers and cathode were sintered at 1565 °C to obtain an efficient attachment between the cathode and the electrolyte and to afford a sintered porous cathode with an electrolyte layer.

Prior to be used, the NiO species have been reduced under hydrogen to form $Ni^+$. This activation step is carried out in a 25 vol.% $H_2$ atmosphere in argon at 700°C for 10 hours, optionally with a pre-calcination at 700°C under pure argon

for 10 hours before the activation step.

Then, an anodic layer was coated on the electrolyte layer of the sintered porous cathode.

The anodic layer is an anode of stainless steel, with channels and a dehydrogenation catalyst comprised within said channels.

The sintered porous cathode with an electrolyte layer was covered with copper, using ion-sputtering. The ion-sputtering conditions involved a Pfeiffer Classic 250 deposition system equipped with two RF (13.56 MHz) power sources, each capable of delivering up to 25 W of power. The system utilizes a Cu target for the deposition process and operates at room temperature. The deposition was carried out under a pure Ar atmosphere with a pressure range of $2.6.10^{-2}$ to $7.4.10^{-2}$ mbar.

A channeled support in steel comprising a dehydrogenation catalyst was then prepared by deep drawing or machining. The fact that deep drawing or machining can be used to prepare the channeled support is advantageous in the sense that it avoids the use of the 3D printing technique. A steel plate and quartz wool was used around the catalyst channels to avoid the catalyst to move. GM31-107 glass sealant tape (commercially available at Schott AG) was used to seal the steel plate to the steel support. The glass sealing tape was activated at 700°C for 2 hours.

Then, the channels of the support were filled by hands with 1.25 g of catalyst powder, namely of PtSnEu support on $Al_2O_3$ (0.5 wt.% of Pt, 2 wt.% of Eu and 3 wt.% of Sn).

The sintered porous cathode with an electrolyte layer covered with copper was thus placed on top of the channeled support comprising the dehydrogenation catalyst and sealed with either GL1734 glass sealing paste (commercially available at Mo-Sci Corporation) or Cotronics Resbond® 908 paste (*i.e.,* an alumina-based bonding ceramic cement) to obtain a proton-conducting catalytic membrane. The glass sealing paste was activated at 620°C for 4, hours, while the Cotronics tape was cured 24 h at room temperature with applied weight.

Finally, an upper steel piece with channels was added to the channeled support and proton-conducting catalytic membrane. Then both steel pieces were sealed together using either GL1734 glass sealing paste (commercially available at Mo-Sci Corporation) or Cotronics Resbond® 908 paste (*i.e.,* an alumina-based bonding ceramic cement) to obtain a proton-conducting catalytic membrane. The glass sealing paste was activated at 620°C for 4 hours, while the Cotronics tape was cured 24 h at room temperature with applied weight.

A closed membrane reactor was thus obtained (see **figures 27** and **28**). As the steel anode comprises the channels, the risks of leaks are decreased once the reactor has been completed. The shape of the steel anode allows for having electrical connections on the side of the membrane, which facilitates their access. This is advantageous in comparison with a reactor assembly with a reactor housing made of Inconel, since in this case, the electrical connections cannot be in contact with the Inconel to ensure an adequate connection.

**Used of a feed comprising one or more vegetable oils and/or one or more triglycerides**

Tables 8 and 9 indicate results involving a feed of vegetable oils with a proton-conducting catalytic membrane operating respectively at a conversion rate of 60% with a 100% extraction ratio and at a conversion rate of 76% with a 90% extraction ratio. The amount of matter that is formed from the propane and that is found in stream 13 is the same for either hydrogen or propylene because it has been generated from the same propane.

Table 8: Simulation at a conversion rate of 60% with a 100% extraction ratio. SOR stands for start-of-run, EOR stands for end-of-run. The last line expresses the results after a simulation of 8000 hours.

| Feed | Rapeseed | | Sunflower | | Corn | |
|---|---|---|---|---|---|---|
| | SOR | EOR | SOR | EOR | SOR | EOR |
| Flow upstream of the hydrotreating unit 5 (T/h) | 77.37 | 77.37 | 77.37 | 77.37 | 77.37 | 77.37 |
| $H_2$ stream 3 (wt.%) | 0.0332 | 0.0292 | 0.036 | 0.032 | 0.036 | 0.032 |
| Feed 1 wt% | 1 | 1 | 1 | 1 | 1 | 1 |
| $H_2$ stream 3 (T/h) | 2.486144 | 2.195107 | 2.688533 | 2.39907 | 2.688533 | 2.39907 |
| Feed 1 (T/h) | 74.88386 | 75.17489 | 74.68147 | 74.97093 | 74.68147 | 74.97093 |
| | | | | | | |
| Propane in effluent 6 (wt.%) | 4.626403 | 4.566654 | 4.633205 | 4.651163 | 4.633205 | 4.651163 |
| Propane in effluent 6 (T/h) | 3.579448 | 3.53322 | 3.58471 | 3.598605 | 3.58471 | 3.598605 |
| Propane in effluent 6 (kmol/h) | 81.16663 | 80.11837 | 81.28595 | 81.60101 | 81.28595 | 81.60101 |

(continued)

| Feed | Rapeseed | | Sunflower | | Corn | |
|---|---|---|---|---|---|---|
| | SOR | EOR | SOR | EOR | SOR | EOR |
| | | | | | | |
| Yield (%) of membrane in the dehydrogenation reactor 15 | 60 | 60 | 60 | 60 | 60 | 60 |
| amount of matter that is formed from the propane and found in stream 13 (kmol/h) | 48.69998 | 48.07102 | 48.77157 | 48.96061 | 48.77157 | 48.96061 |
| Propylene in first effluent 19 (T/h) | 2.049295 | 2.022829 | 2.052308 | 2.060262 | 2.052308 | 2.060262 |
| Hydrogen in second effluent 17 (kg/h) | 97.88695 | 96.62275 | 98.03086 | 98.41082 | 98.03086 | 98.41082 |
| | | | | | | |
| Hydrogen recycled in second effluent 17 (wt.% based on the total weight of the hydrogen stream 3) | 3.9373 | 4.401733 | 3.646259 | 4.102041 | 3.646259 | 4.102041 |
| | | | | | | |
| Hydrogen (T/y) | 783.0956 | 772.982 | 784.2469 | 787.2866 | 784.2469 | 787.2866 |

Table 8 - sequel

| Feed | Soya | | Carinata | |
|---|---|---|---|---|
| | SOR | EOR | SOR | EOR |
| Flow upstream of the hydrotreating unit 5 (T/h) | 77.37 | 77.37 | 77.37 | 77.37 |
| $H_2$ stream 3 (wt.%) | 0.036 | 0.032 | 0.035 | 0.031 |
| Feed 1 wt% | 1 | 1 | 1 | 1 |
| $H_2$ stream 3 (T/h) | 2.688533 | 2.39907 | 2.616377 | 2.326353 |
| Feed 1 (T/h) | 74.68147 | 74.97093 | 74.75362 | 75.04365 |
| | | | | |
| Propane in effluent 6 (wt.%) | 4.633205 | 4.651163 | 4.444444 | 4.461688 |
| Propane in effluent 6 (T/h) | 3.58471 | 3.598605 | 3.438667 | 3.452008 |
| Propane in effluent 6 (kmol/h) | 81.28595 | 81.60101 | 77.9743 | 78.27682 |
| | | | | |
| Yield (%) of membrane in the dehydrogenation reactor 15 | 60 | 60 | 60 | 60 |
| amount of matter that is formed from the propane and found in stream 13 (kmol/h) | 48.77157 | 48.96061 | 46.78458 | 46.96609 |
| Propylene in first effluent 19 (T/h) | 2.052308 | 2.060262 | 1.968695 | 1.976333 |
| Hydrogen in second effluent 17 (kg/h) | 98.03086 | 98.41082 | 94.03701 | 94.40184 |
| | | | | |
| Hydrogen recycled in second effluent 17 (wt.% based on the total weight of the hydrogen stream 3) | 3.646259 | 4.102041 | 3.594169 | 4.057933 |
| | | | | |
| Hydrogen (T/y) | 784.2469 | 787.2866 | 752.2961 | 755.2148 |

Table 9: Simulation at a conversion rate of 76% with a 90% extraction ratio. SOR stands for start-of-run, EOR stands for end-of-run. The last line expresses the results after a simulation of 8000 hours.

| Feed | Rapeseed | | Sunflower | | Corn | |
|---|---|---|---|---|---|---|
| | SOR | EOR | SOR | EOR | SOR | EOR |
| Flow upstream of the hydrotreating unit 5 (T/h) | 77.37 | 77.37 | 77.37 | 77.37 | 77.37 | 77.37 |
| $H_2$ stream 3 (wt.%) | 0.0332 | 0.0292 | 0.036 | 0.032 | 0.036 | 0.032 |
| Feed 1 wt% | 1 | 1 | 1 | 1 | 1 | 1 |
| $H_2$ stream 3 (T/h) | 2.486144 | 2.195107 | 2.688533 | 2.39907 | 2.688533 | 2.39907 |
| Feed 1 (T/h) | 74.88386 | 75.17489 | 74.68147 | 74.97093 | 74.68147 | 74.97093 |
| | | | | | | |
| Propane in effluent 6 (wt.%) | 4.626403 | 4.566654 | 4.633205 | 4.651163 | 4.633205 | 4.651163 |
| Propane in effluent 6 (T/h) | 3.579448 | 3.53322 | 3.58471 | 3.598605 | 3.58471 | 3.598605 |
| Propane in effluent 6 (kmol/h) | 81.16663 | 80.11837 | 81.28595 | 81.60101 | 81.28595 | 81.60101 |
| | | | | | | |
| Yield (%) of membrane in the dehydrogenation reactor 15 | 76 | 76 | 76 | 76 | 76 | 76 |
| amount of matter that is formed from the propane and found in stream 13 (kmol/h) | 61.68664 | 60.88996 | 61.77732 | 62.01677 | 61.77732 | 62.01677 |
| Propylene in first effluent 19 (T/h) | 2.595774 | 2.562249 | 2.59959 | 2.609666 | 2.59959 | 2.609666 |
| Hydrogen in second effluent 17 (kg/h) | 111.5911 | 110.1499 | 111.7552 | 112.1883 | 111.7552 | 112.1883 |
| | | | | | | |
| Hydrogen recycled in second effluent 17 (wt.% based on the total weight of the hydrogen stream 3) | 4.488522 | 5.017976 | 4.156735 | 4.676327 | 4.156735 | 4.676327 |
| | | | | | | |
| Hydrogen (T/y) | 892.729 | 881.1995 | 894.0414 | 897.5067 | 894.0414 | 897.5067 |

Table 9 - sequel

| Feed | Soya | | Carinata | |
|---|---|---|---|---|
| | SOR | EOR | SOR | EOR |
| Flow upstream of the hydrotreating unit 5 (T/h) | 77.37 | 77.37 | 77.37 | 77.37 |
| $H_2$ stream 3 (wt.%) | 0.036 | 0.032 | 0.035 | 0.031 |
| Feed 1 wt% | 1 | 1 | 1 | 1 |
| $H_2$ stream 3 (T/h) | 2.688533 | 2.39907 | 2.616377 | 2.326353 |
| Feed 1 (T/h) | 74.68147 | 74.97093 | 74.75362 | 75.04365 |
| | | | | |
| Propane in effluent 6 (wt.%) | 4.633205 | 4.651163 | 4.444444 | 4.461688 |
| Propane in effluent 6 (T/h) | 3.58471 | 3.598605 | 3.438667 | 3.452008 |
| Propane in effluent 6 (kmol/h) | 81.28595 | 81.60101 | 77.9743 | 78.27682 |
| | | | | |
| Yield (%) of membrane in the dehydrogenation reactor 15 | 76 | 76 | 76 | 76 |

(continued)

| Feed | Soya | | Carinata | |
|---|---|---|---|---|
| | SOR | EOR | SOR | EOR |
| amount of matter that is formed from the propane and found in stream 13 (kmol/h) | 61.77732 | 62.01677 | 59.26047 | 59.49038 |
| Propylene in first effluent 19 (T/h) | 2.59959 | 2.609666 | 2.493681 | 2.503355 |
| Hydrogen in second effluent 17 (kg/h) | 111.7552 | 112.1883 | 107.2022 | 107.6181 |
| | | | | |
| Hydrogen recycled in second effluent 17 (wt.% based on the total weight of the hydrogen stream 3) | 4.156735 | 4.676327 | 4.097353 | 4.626043 |
| | | | | |
| Hydrogen (T/y) | 894.0414 | 897.5067 | 857.6175 | 860.9448 |

The results indicated on tables 8 and 9 show that vegetables oils can be used as a source of green propylene and that hydrogen can be recycled, in an amount ranging between 3 wt.% and 10 wt.%, or between 3.5 wt.% and 5.5 wt% of the total weight of the hydrogen stream that is used to hydrogenate and/or hydrotreat the feed comprising said vegetable oils.

## Claims

**1.** A propane dehydrogenation process is **characterized in that** it comprises the following steps:

a) providing a feed (1) comprising one or more vegetable oils;
b) providing an hydrogen stream (3) and performing an hydrogenation reaction on said feed (1) to generate at least one effluent (6) comprising propane and one or more fatty acids;
c) separating propane from said one or more fatty acids comprised within said at least one effluent (6)generated at step (b) to generate a stream (13) comprising propane;
d) providing at least one proton-conducting catalytic membrane, each proton-conducting catalytic membrane comprising at least one dehydrogenation catalyst;
e) feeding to said one or more proton-conducting catalytic membranes under propane dehydrogenation conditions the stream (13) generated at step (c);
f) recovering a first effluent (19) comprising at least propylene;
g) optionally, recovering a second effluent (17) comprising hydrogen.

**2.** The propane dehydrogenation process according to claim 1, **characterized in that** the feed (1) comprising one or more vegetable oils comprises one or more triglycerides; with preference, each triglyceride has three aliphatic chains and the process is **characterized in that** at least two of the three aliphatic chains are identical and have a number of carbons ranging between 7 and 22.

**3.** The propane dehydrogenation process according to claim 1 or 2, **characterized in that** the hydrogenation reaction performed in step (b) is carried out at a flow of at most 200 T/hour.

**4.** The propane dehydrogenation process according to any one of claims 1 to 3, wherein step (g) is carried out, is **characterized in that** the second effluent (17) recovered is used at least partially in the hydrogenation reaction performed at step (b); with preference, the process is **characterized in that** the part of the second effluent (17) that is used in said hydrogenation reaction amounts to between 3 wt.% and 10 wt.% of the total weight of the hydrogen stream (3).

**5.** The propane dehydrogenation process according to any one of claims 1 to 4, wherein step (g) is carried out, is **characterized in that** the second effluent (17) recovered is used at least partially during a hydrotreating process of the feed (1) provided at step (a); with preference, said hydrotreating process comprises the hydrogenation reaction of the feed (1) performed at step (b).

**6.** The propane dehydrogenation process according to any one of claims 1 to 5, wherein step (g) is carried out, is

**characterized in that** the second effluent (17) recovered is used at least partially during a hydroizomerisation process of one or more hydrotreated oils; with preference, said one or or more hydrotreated oils are generated from a hydrotreating process of the feed (1) and said hydrotreating process comprises the hydrogenation of the feed (1) performed at step (b).

**7.** The propane dehydrogenation process according to any one of claims 1 to 6, **characterized in that** each proton-conducting catalytic membrane comprises an anode, an electrolyte layer disposed on top of the anode and a porous cathode disposed on top of the electrolyte layer, wherein said at least one dehydrogenation catalyst is comprised within the anode.

**8.** The propane dehydrogenation process according to claim 7, **characterized in that** the anode comprises one or more channels, each of said channels comprising said at least one dehydrogenation catalyst; with preference, said one or more channels are made in copper or steel.

**9.** The propane dehydrogenation process according to claim 8, **characterized in that** said one or more channels are flared depth channels or rectangular channels; with preference, said one or more channels are flared depth channels.

**10.** The propane dehydrogenation process according to any one of claims 1 to 9 is **characterized in that** the one or more dehydrogenation catalysts comprise one or more metal-based catalysts, one or more metal oxide-based catalysts, one or more zeolites, one or more transition metal carbides, one or more transition metal nitrides, one or more carbon nanotubes, or any mixtures thereof.

**11.** The propane dehydrogenation process according to any one of claims 1 to 10 is **characterized in that** the one or more proton-conducting catalytic membranes provided at step (d) are one or more co-ionic catalytic membranes.

**12.** The propane dehydrogenation process according to any one of claims 1 to 11 is **characterized in that** said one or more proton-conducting catalytic membranes are selected from one or more palladium membranes, one or more palladium-silver alloy membranes, one or more silica/alumina membranes, one or more zeolite-based membranes, one or more ceramic membranes, one or more ceramic electrolytic membranes, one or more polymeric membranes or any combinations thereof.

**13.** The propane dehydrogenation process according to any one of claims 1 to 12 is **characterized in that** the propane dehydrogenation conditions of step (e) comprise a temperature ranging between 425°C and 575°C **and/or** a space velocity of at least 150 Nml/h/g.

**14.** The propane dehydrogenation process according to any one of claims 1 to 13 is **characterized in that** it comprises a step of providing steam in a pulsed manner into the stream (13) comprising propane generated at step (c) and/or **in that** the stream (13) comprising propane generated at step (c) further comprises steam in an amount ranging between 1 vol.% and 15 vol.% of the total volume of the stream (13) comprising propane generated at step (c).

**15.** Installation for performing a propane dehydrogenation process, said installation comprising at least one dehydrogenation reactor (15) comprising at least one dehydrogenation catalyst and is **characterized in that** at least one hydrotreating unit (5) is placed upstream of said one or more dehydrogenation reactors (15), with a propane-separation unit (7) being placed between said one or more hydrotreating unit (5) and said one or more dehydrogenation reactors (15), and wherein the one or more hydrotreating unit (5), the one or more propane-separation unit (7) and the one or more dehydrogenation reactors (15) are in fluidic connection; with preference, said propane-separation unit (7) comprises a separator (9) and/or a distillation column (11).

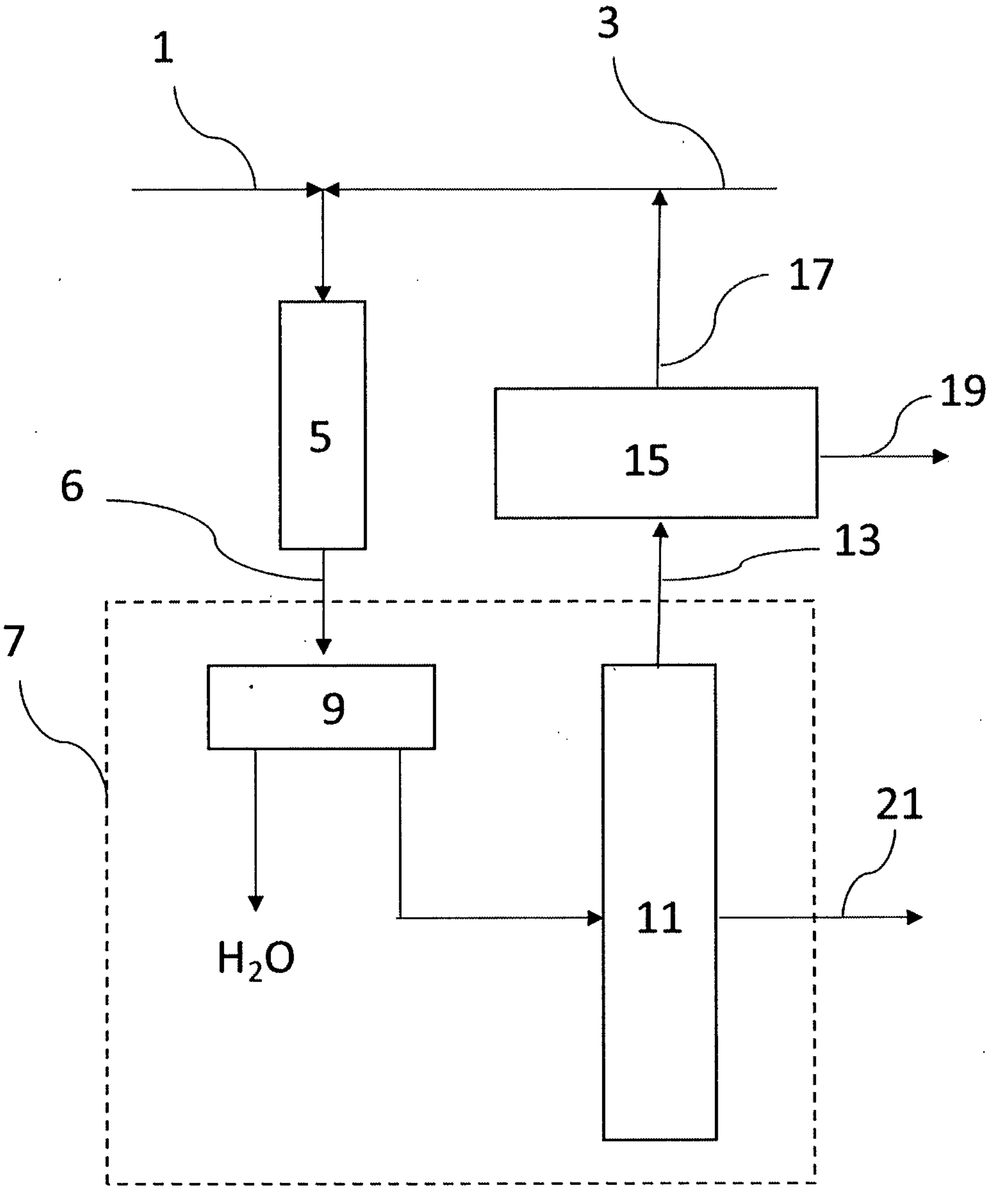


Fig. 1

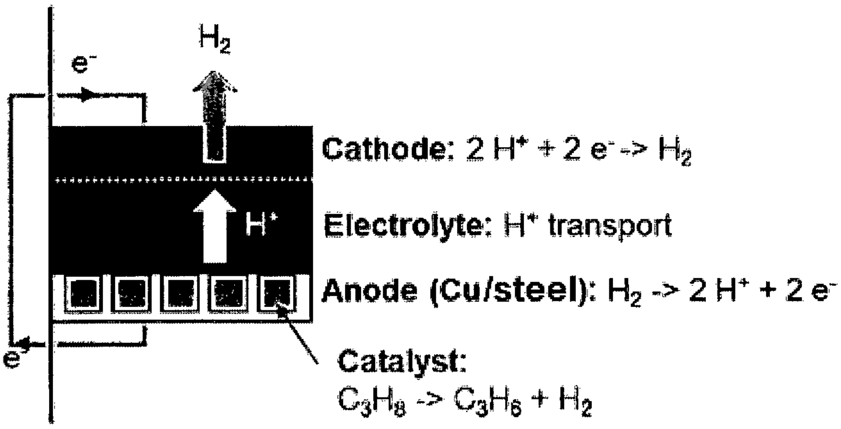
H2
e-
Cathode: 2 H+ + 2 e- -> H2
H+
Electrolyte: H+ transport
Anode (Cu/steel): H2 -> 2 H+ + 2 e-
e-
Catalyst:
C3H8 -> C3H6 + H2

Fig. 2

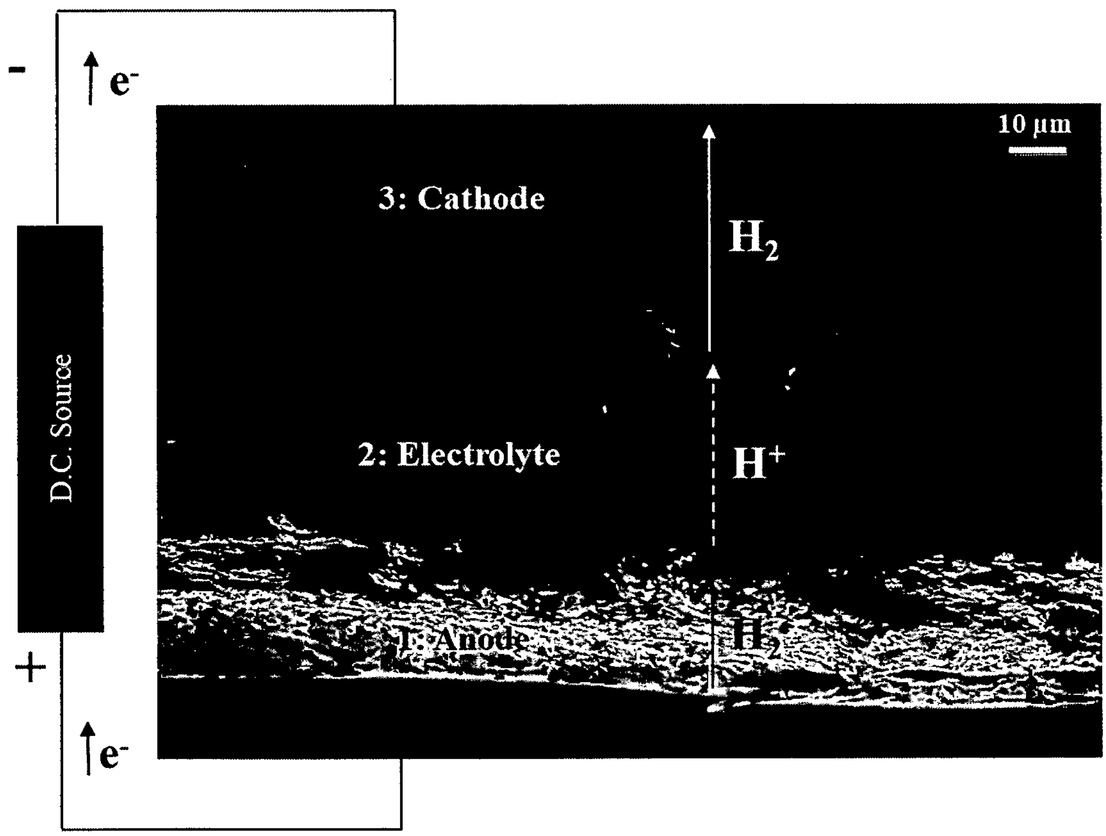
-
e-
10 µm
3: Cathode
H2
D.C. Source
2: Electrolyte
H+
1: Anode
H2
+
e-

Fig. 3

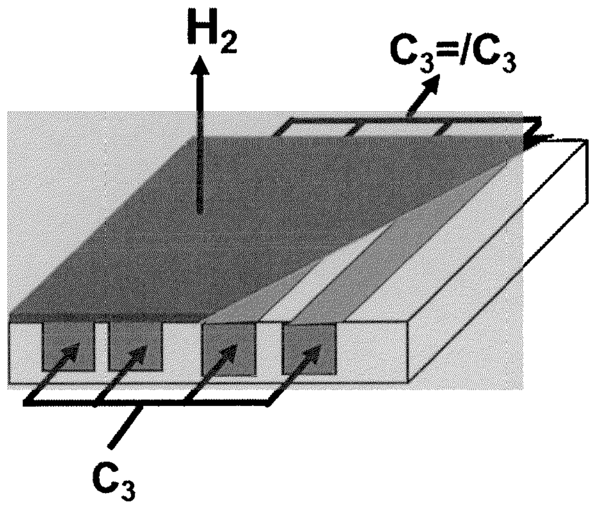


Fig. 4

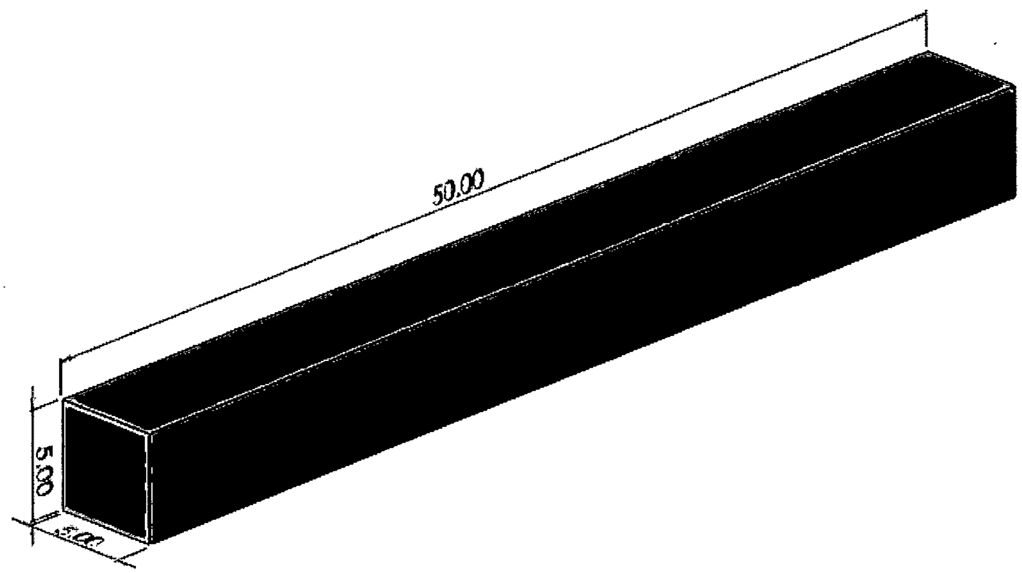


Fig. 5

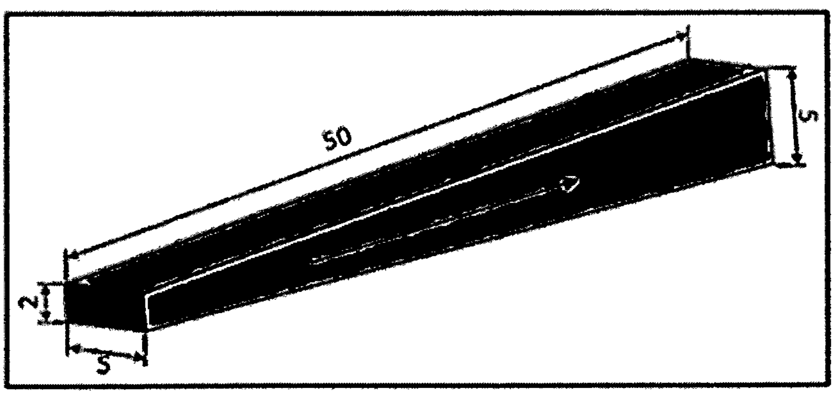


Fig. 6

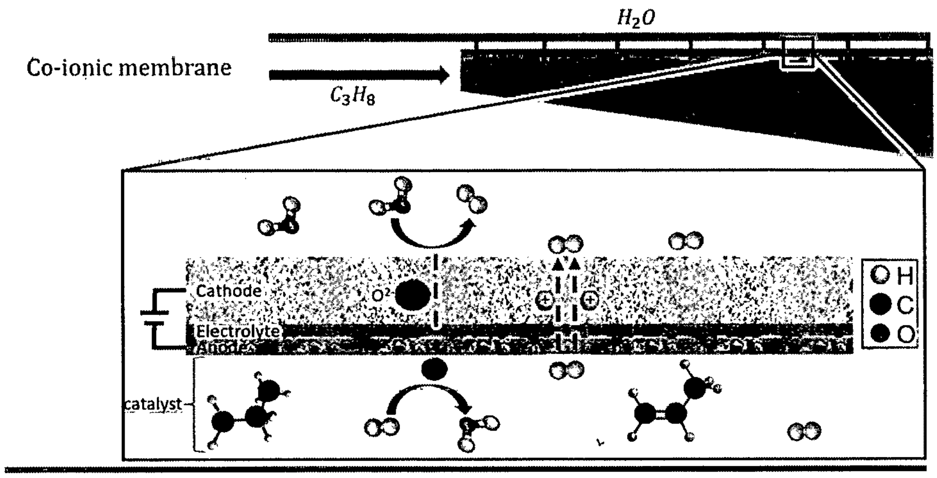
$H_2O$
Co-ionic membrane
$C_3H_8$
Cathode
$O^{2-}$
Electrolyte
Anode
catalyst
H
C
O

Fig. 7

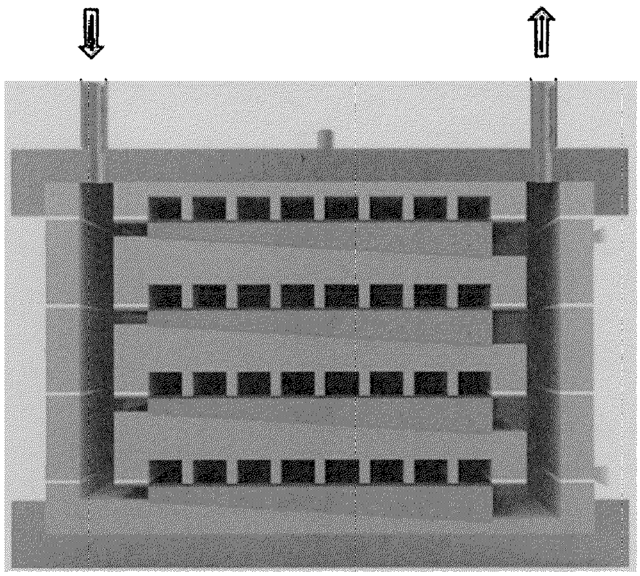

Fig. 8

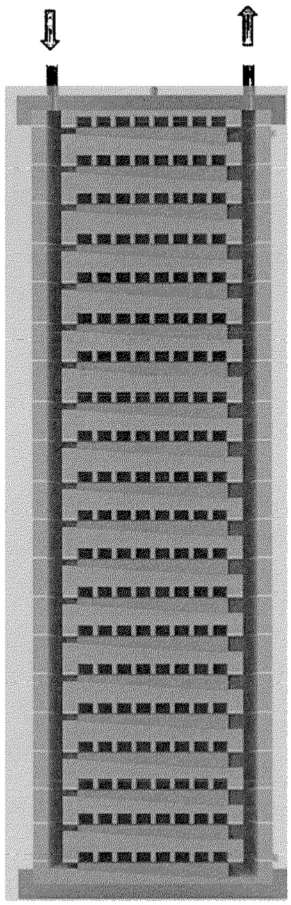

Fig.9

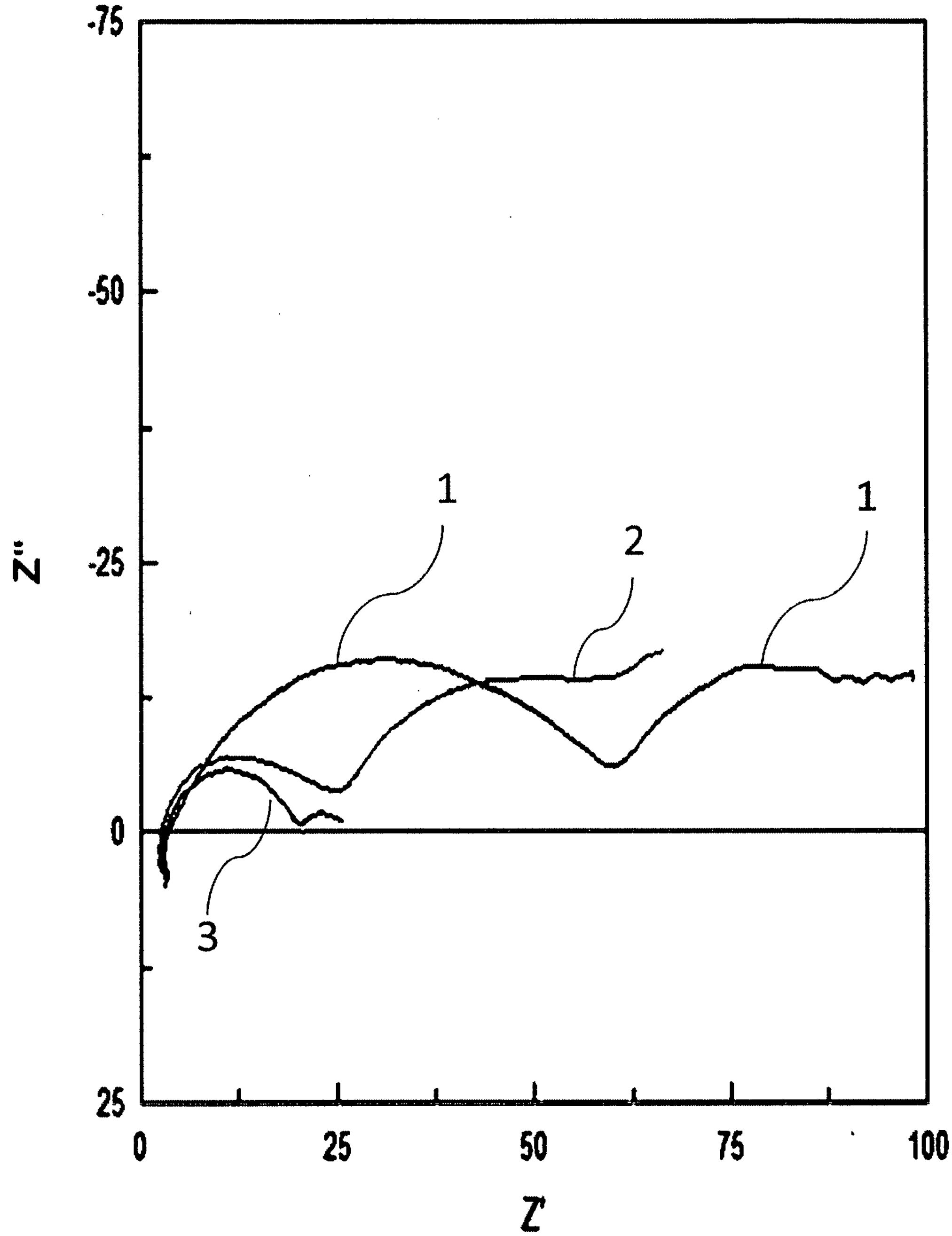


Fig. 10

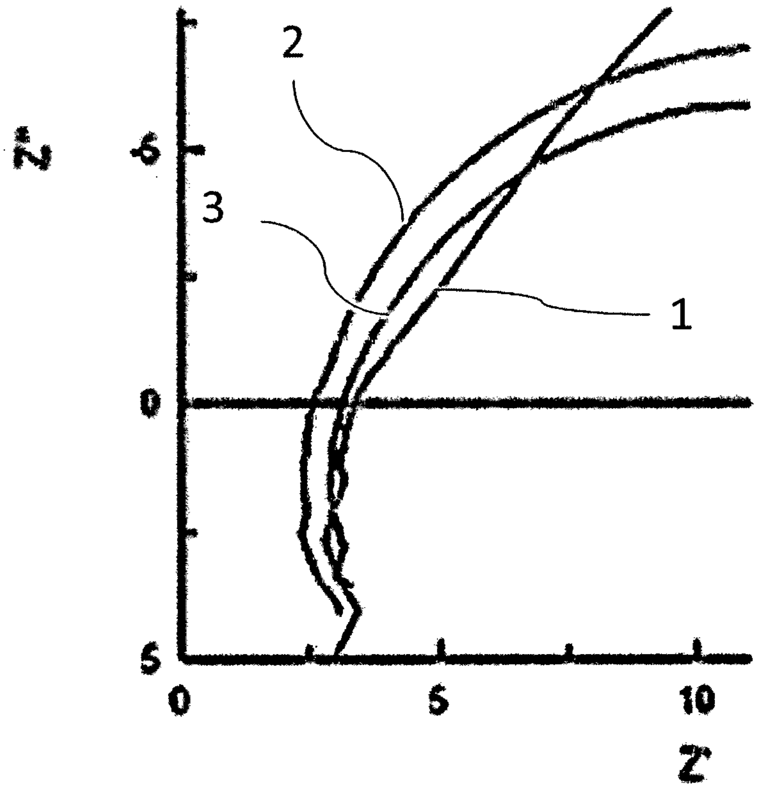


Fig. 11

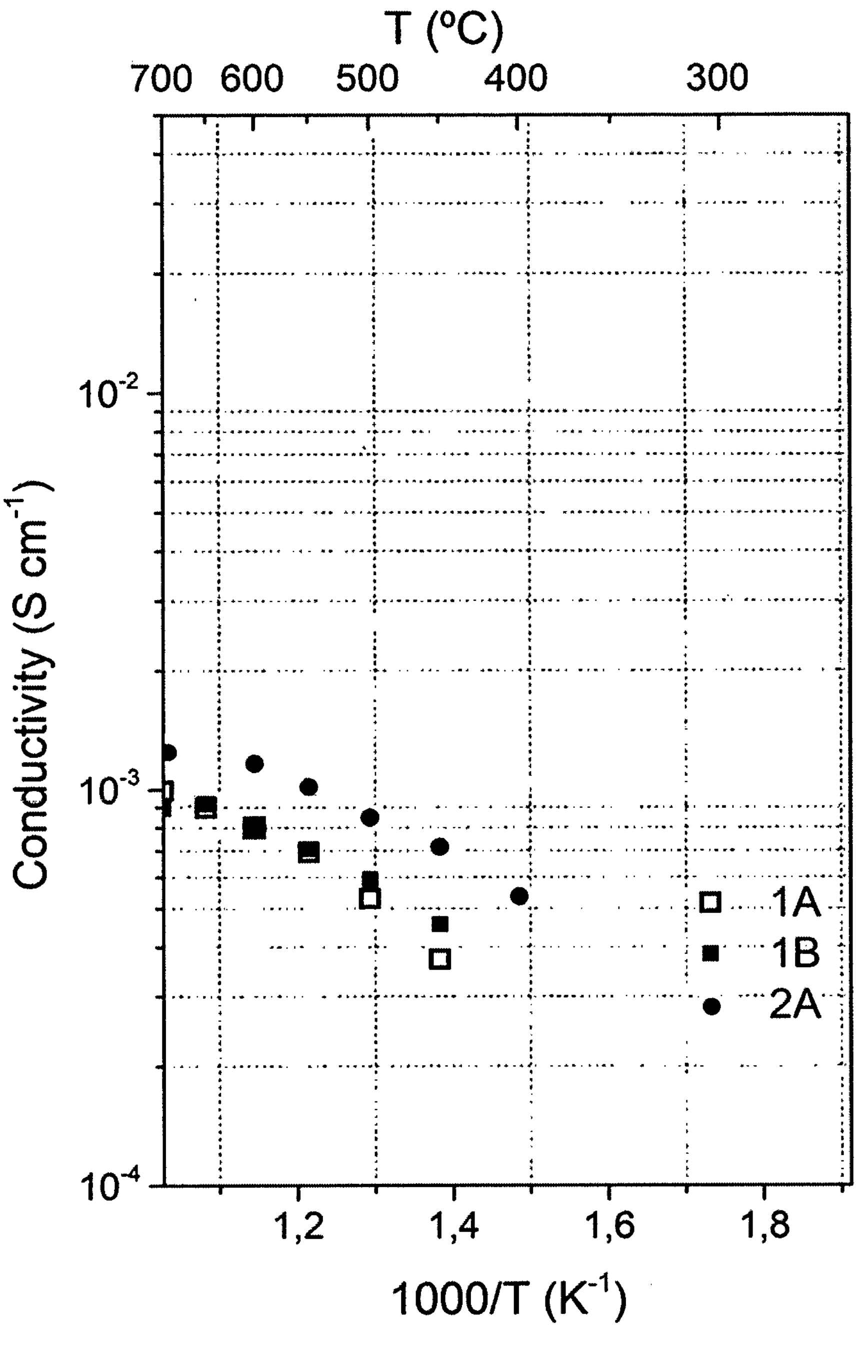
T (ºC)
700 600 500 400 300
Conductivity (S cm-1)
10-2
10-3
10-4
1,2 1,4 1,6 1,8
1000/T (K-1)
1A
1B
2A

Fig. 12

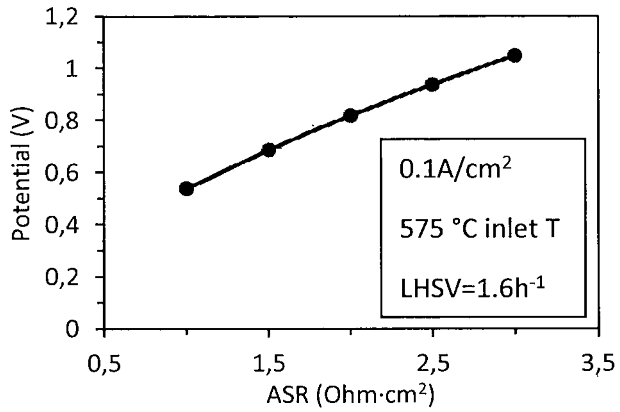
1,2
1
0,8
0,6
0,4
0,2
0
Potential (V)
0,5
1,5
2,5
3,5
ASR (Ohm·cm²)
0.1A/cm²
575 °C inlet T
LHSV=1.6h⁻¹

**Fig. 13**

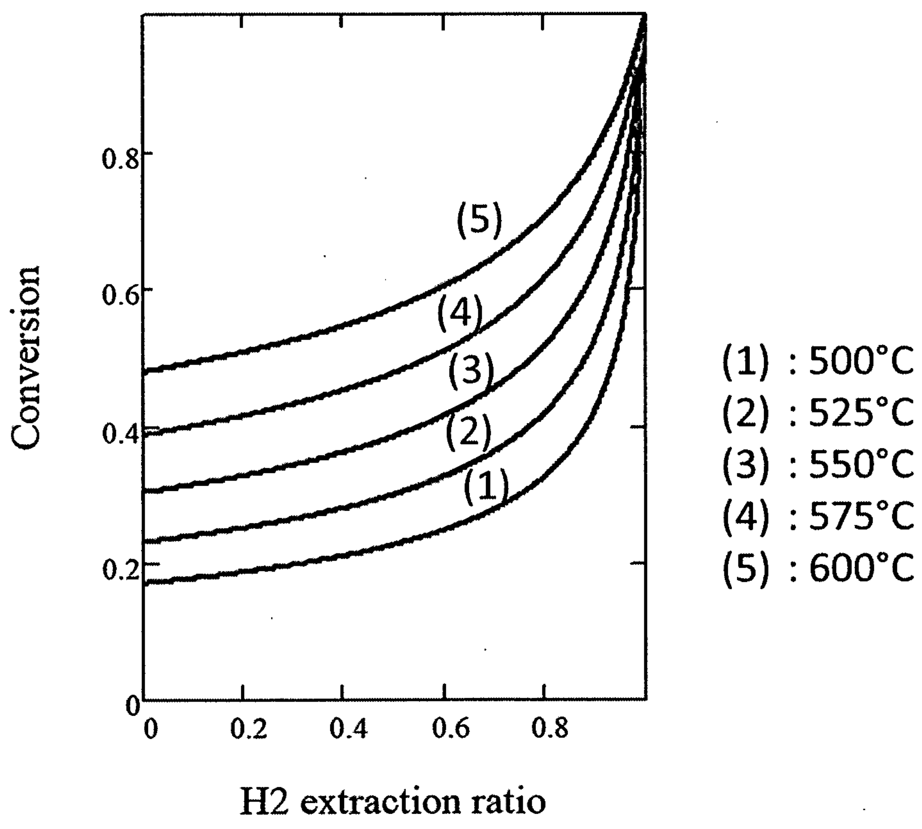
Conversion
0.8
0.6
0.4
0.2
0
0
0.2
0.4
0.6
0.8
H2 extraction ratio
(5)
(4)
(3)
(2)
(1)
(1) : 500°C
(2) : 525°C
(3) : 550°C
(4) : 575°C
(5) : 600°C

**Fig. 14**

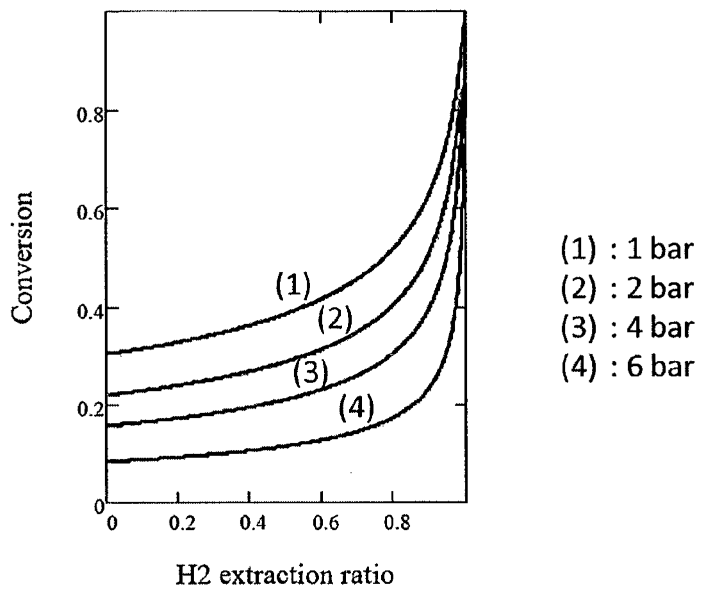
Conversion
0.8
0.6
0.4
0.2
0
0
0.2
0.4
0.6
0.8
H2 extraction ratio
(1)
(2)
(3)
(4)
(1) : 1 bar
(2) : 2 bar
(3) : 4 bar
(4) : 6 bar

Fig. 15

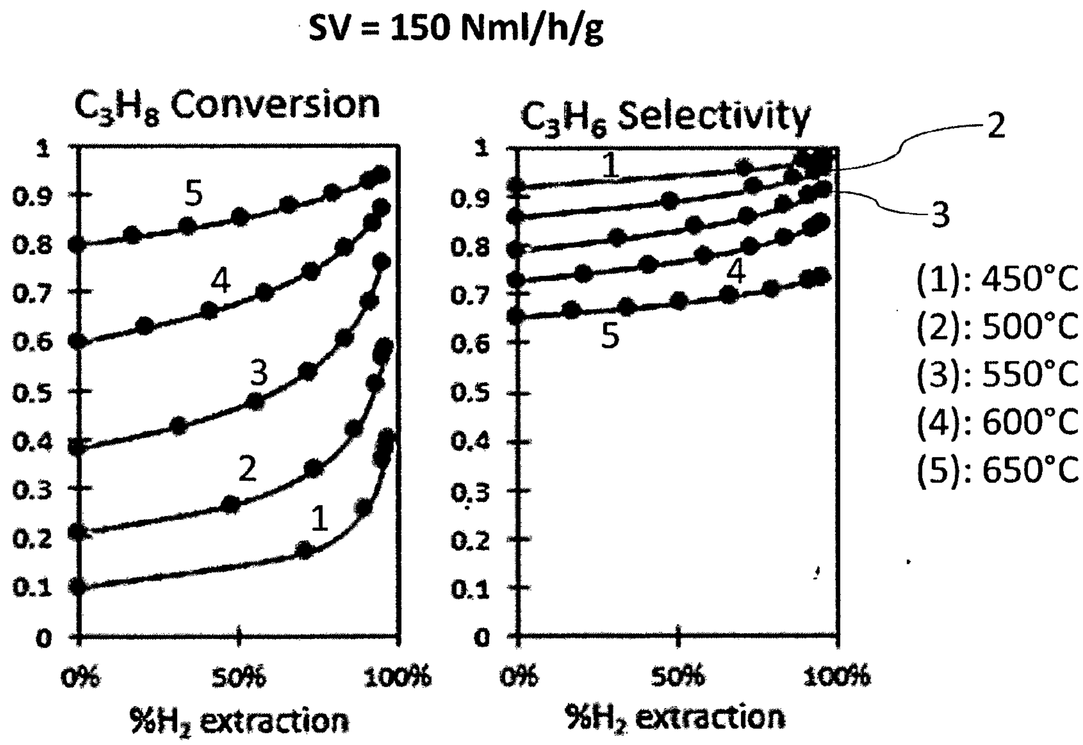
SV = 150 Nml/h/g
$C_3H_8$ Conversion
$C_3H_6$ Selectivity
1
0.9
0.8
0.7
0.6
0.5
0.4
0.3
0.2
0.1
0
0%
50%
100%
$\%H_2$ extraction
1
2
3
4
5
(1): 450°C
(2): 500°C
(3): 550°C
(4): 600°C
(5): 650°C

Fig. 16

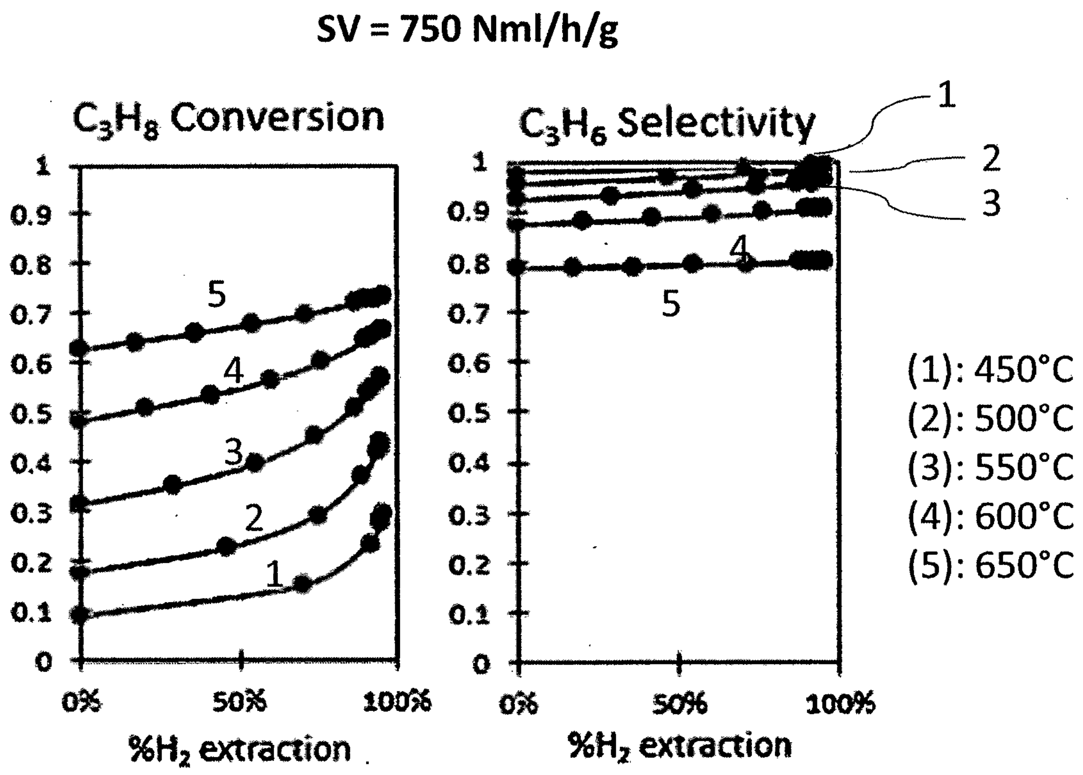
SV = 750 Nml/h/g
$C_3H_8$ Conversion
$C_3H_6$ Selectivity
%$H_2$ extraction
%$H_2$ extraction
(1): 450°C
(2): 500°C
(3): 550°C
(4): 600°C
(5): 650°C

Fig. 17

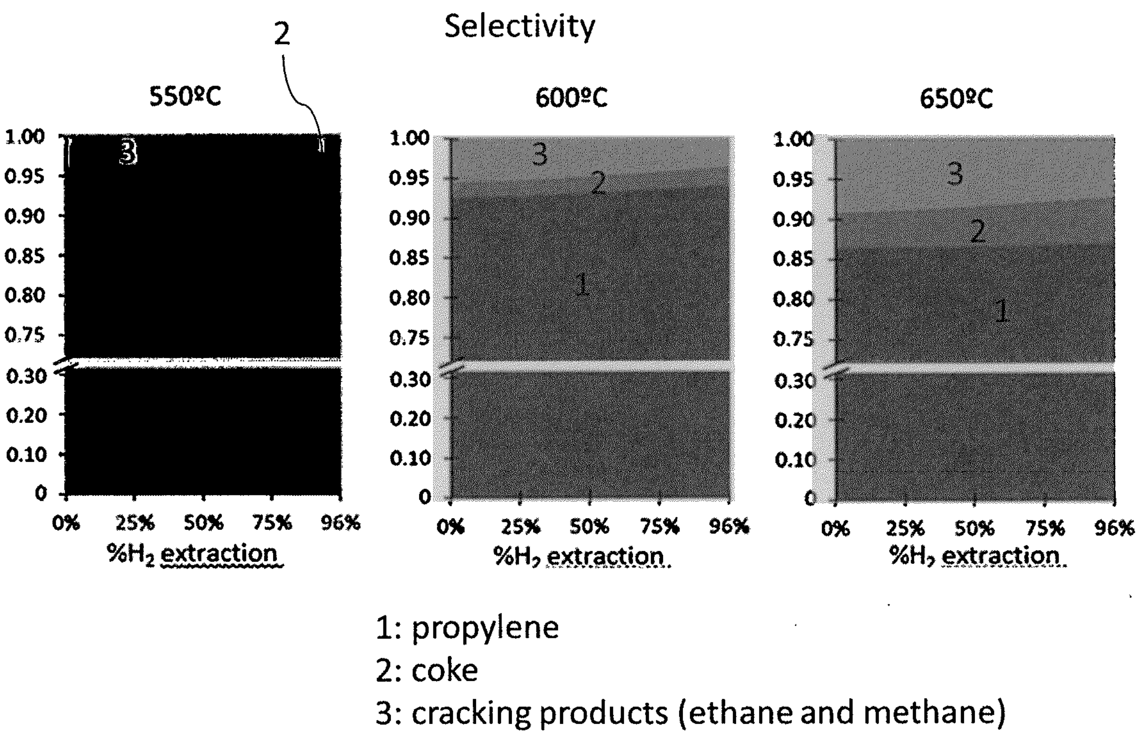
Selectivity
550ºC
600ºC
650ºC
%$H_2$ extraction
%$H_2$ extraction
%$H_2$ extraction
1: propylene
2: coke
3: cracking products (ethane and methane)

Fig. 18

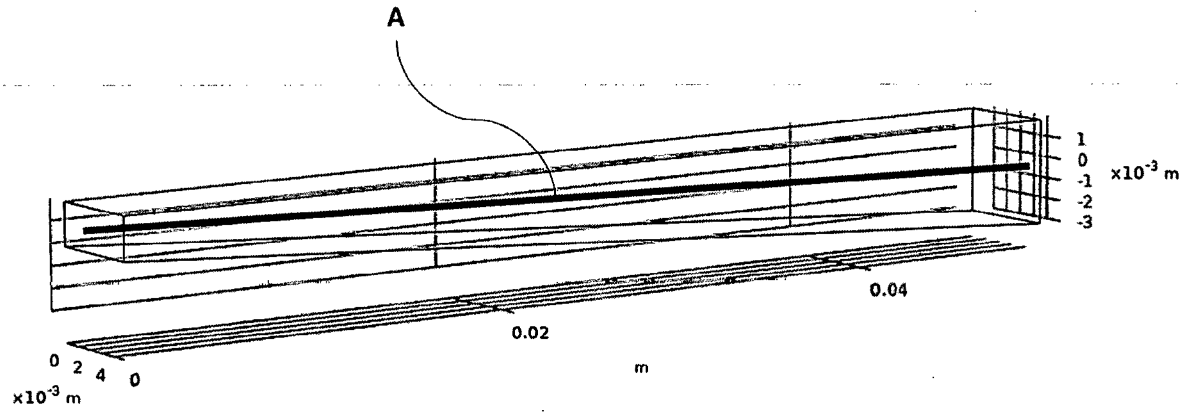
A
1
0
-1
-2
-3
x10-3 m
0.04
0.02
m
0 2 4 0
x10-3 m

Fig .19

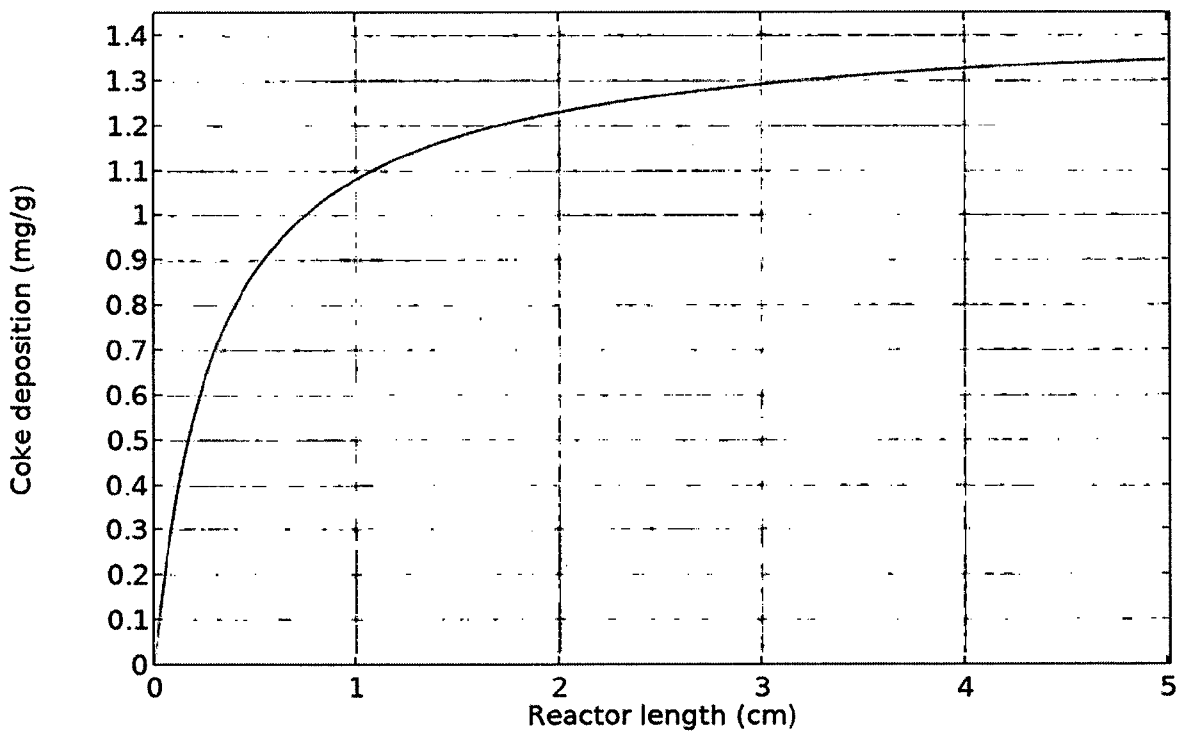
Coke deposition (mg/g)
1.4
1.3
1.2
1.1
1
0.9
0.8
0.7
0.6
0.5
0.4
0.3
0.2
0.1
0
0
1
2
3
4
5
Reactor length (cm)

Fig. 20

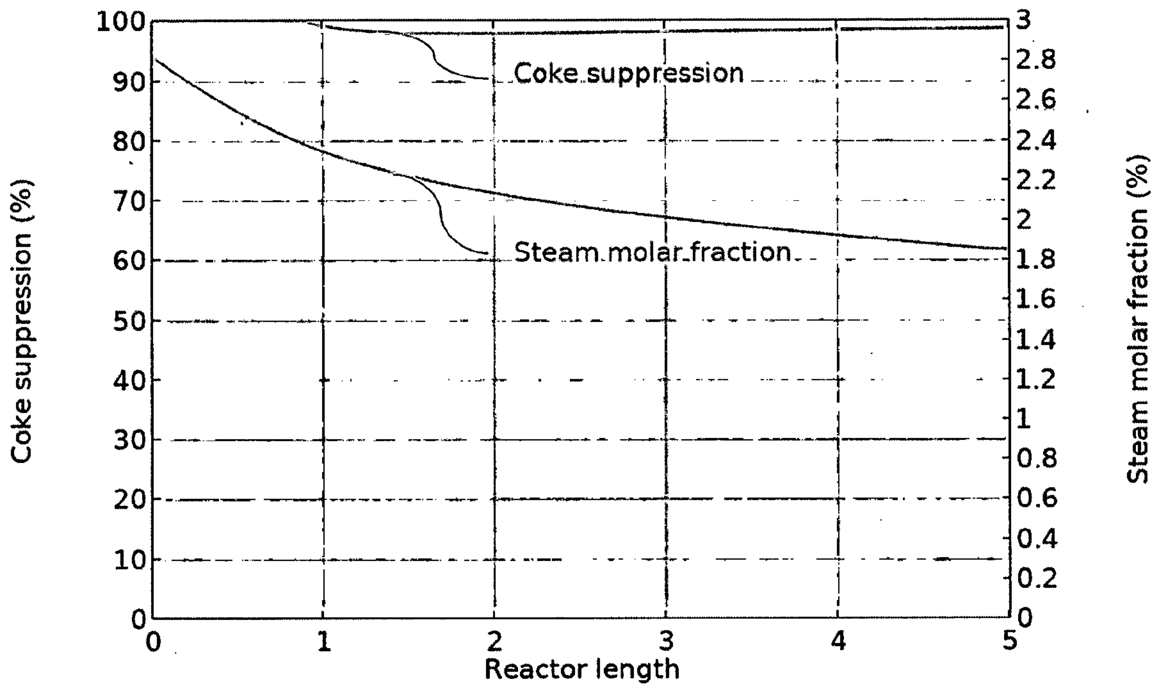
Coke suppression
Steam molar fraction
Coke suppression (%)
Steam molar fraction (%)
Reactor length
100
90
80
70
60
50
40
30
20
10
0
3
2.8
2.6
2.4
2.2
2
1.8
1.6
1.4
1.2
1
0.8
0.6
0.4
0.2
0
1
2
3
4
5

Fig. 21

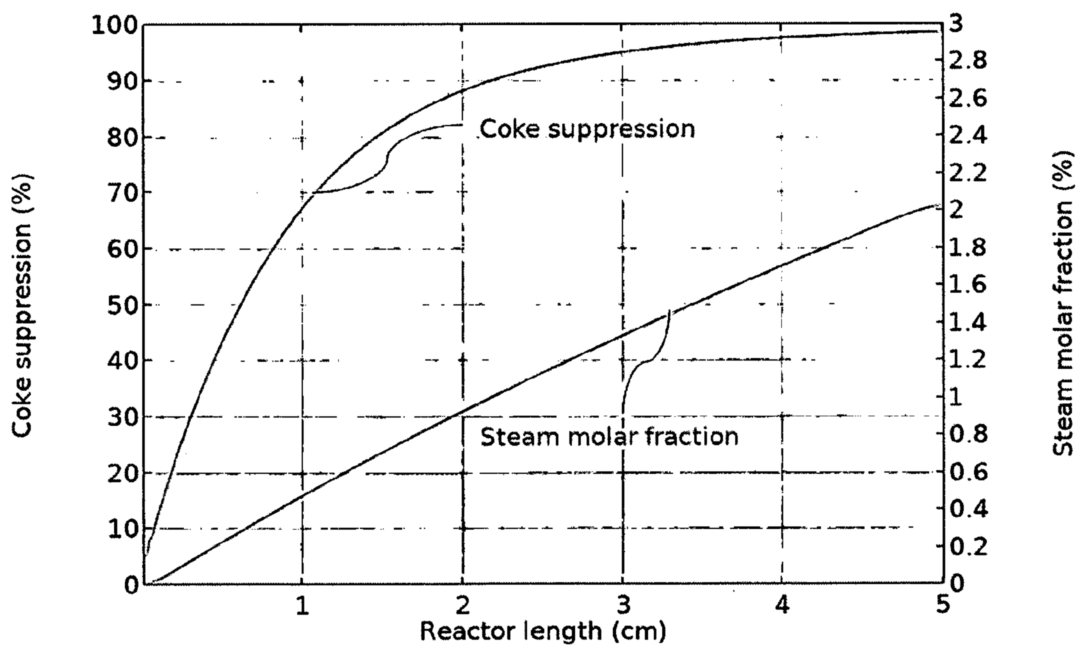
Coke suppression
Steam molar fraction
Coke suppression (%)
Steam molar fraction (%)
Reactor length (cm)
100
90
80
70
60
50
40
30
20
10
0
3
2.8
2.6
2.4
2.2
2
1.8
1.6
1.4
1.2
1
0.8
0.6
0.4
0.2
0
1
2
3
4
5

Fig. 22

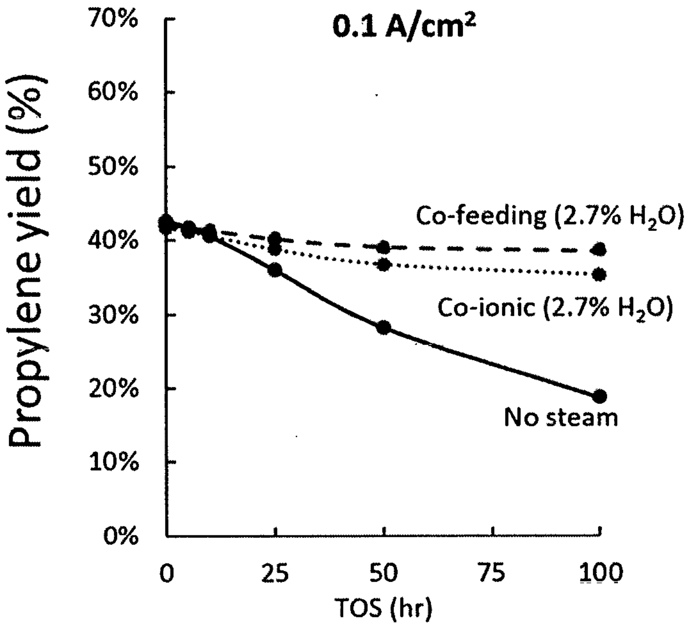
0.1 A/cm²
Propylene yield (%)
70%
60%
50%
40%
30%
20%
10%
0%
Co-feeding (2.7% $H_2O$)
Co-ionic (2.7% $H_2O$)
No steam
0
25
50
75
100
TOS (hr)

Fig. 23

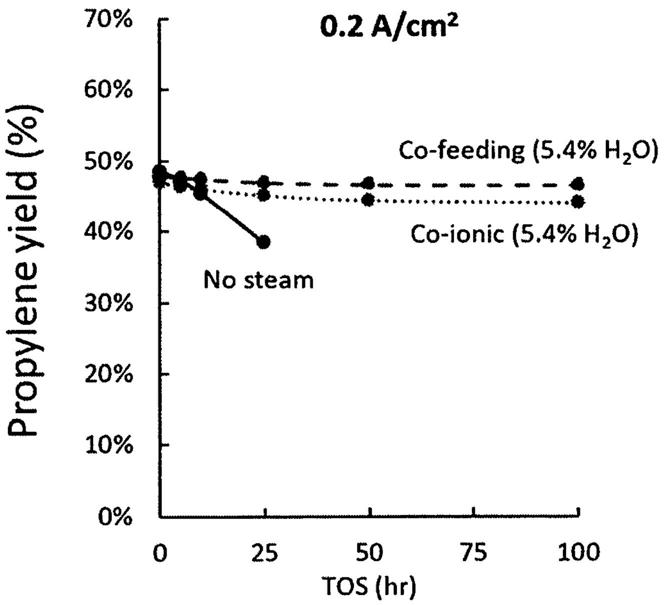
0.2 A/cm²
Propylene yield (%)
70%
60%
50%
40%
30%
20%
10%
0%
Co-feeding (5.4% $H_2O$)
Co-ionic (5.4% $H_2O$)
No steam
0
25
50
75
100
TOS (hr)

Fig. 24

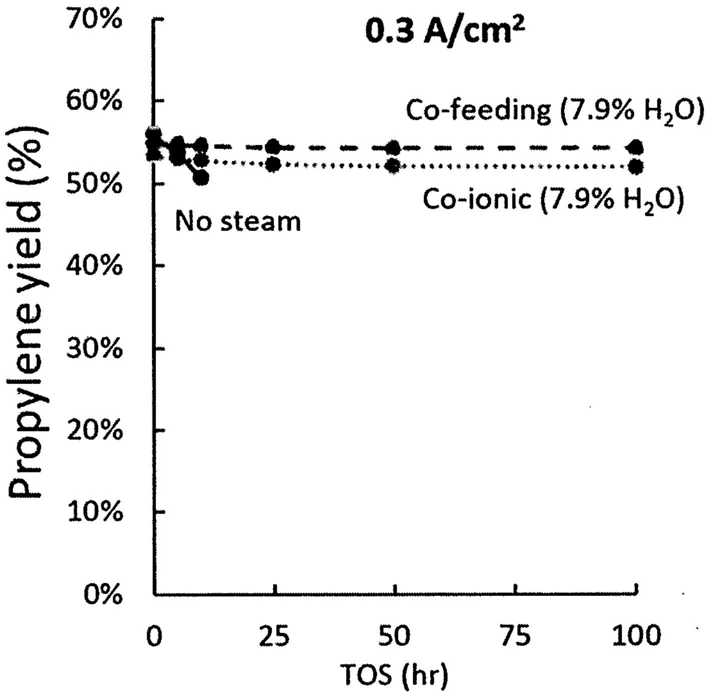
0.3 A/cm²
Co-feeding (7.9% $H_2O$)
Co-ionic (7.9% $H_2O$)
No steam
Propylene yield (%)
70%
60%
50%
40%
30%
20%
10%
0%
0
25
50
75
100
TOS (hr)

Fig. 25

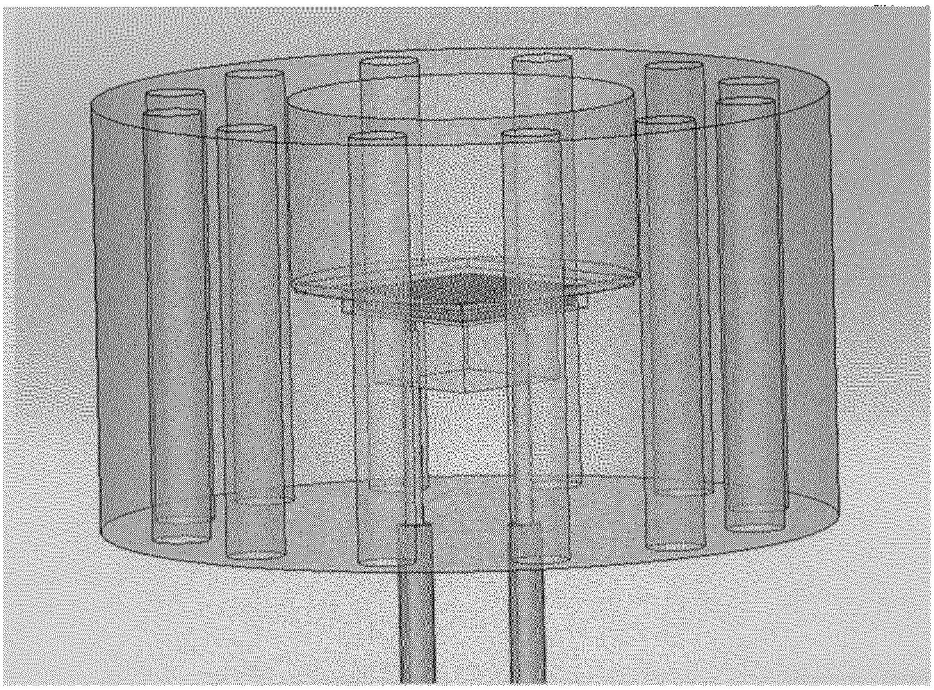

Fig. 26

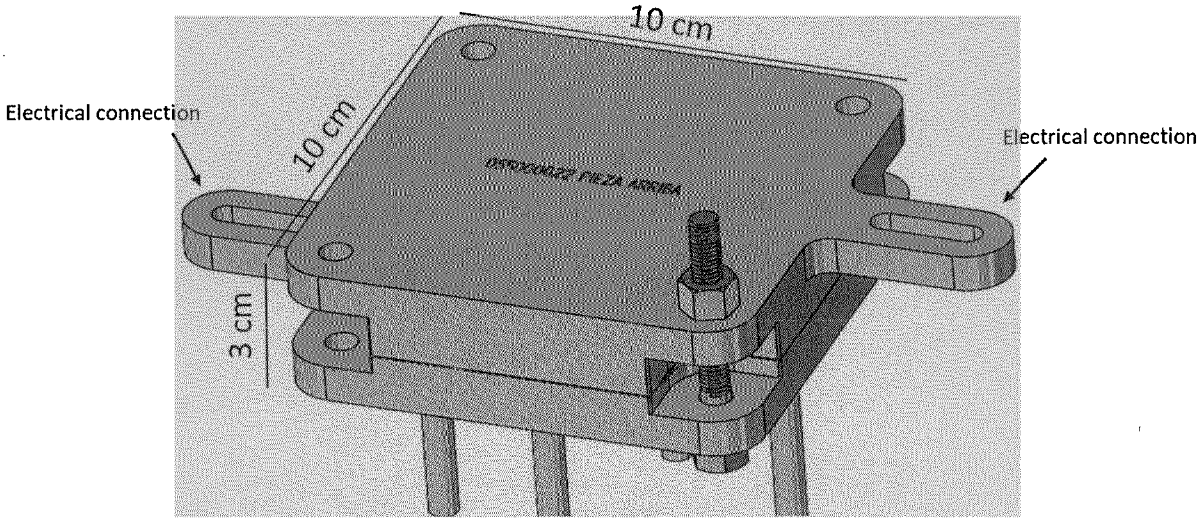
10 cm
10 cm
3 cm
Electrical connection
Electrical connection
055000022 PIEZA ARRIBA

Fig. 27

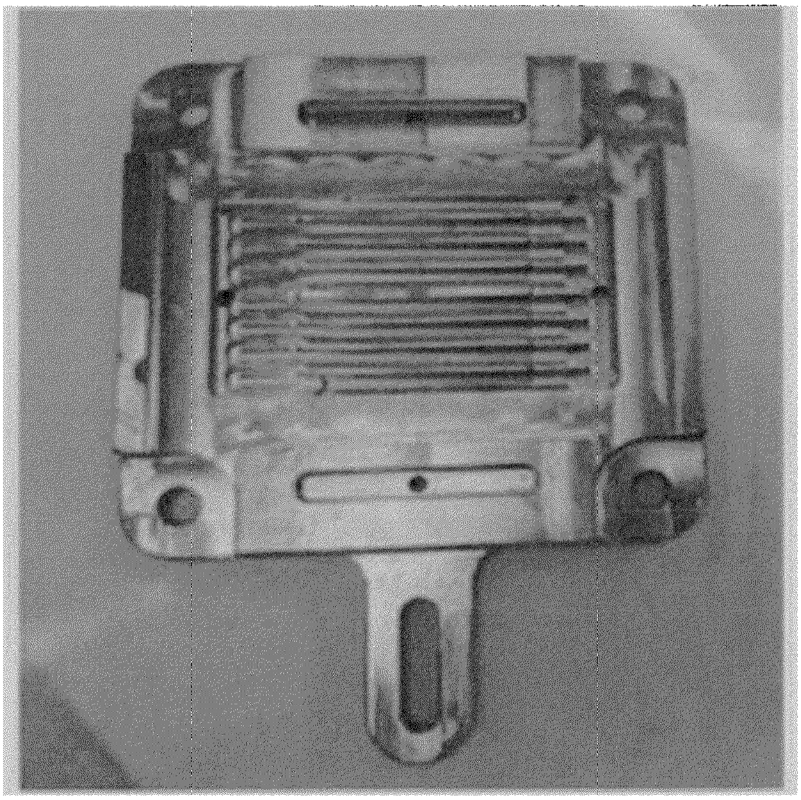

Fig. 28

Europäisches Patentamt
European Patent Office
Office européen des brevets

# EUROPEAN SEARCH REPORT

Application Number
EP 24 31 5572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2023/020748 A1 (OJALA ANTTI [FI] ET AL) 19 January 2023 (2023-01-19)<br>* paragraphs [0001], [044330], [0023], [0270], [0272] *<br>----- | 1,2,4, 10,15<br>1-15 | INV.<br>C07C5/333<br>C07C11/06<br>C10G3/00<br>C25B3/00 |
| Y | EP 2 417 058 A1 (BASF SE [DE]) 15 February 2012 (2012-02-15)<br>* the whole document *<br>* paragraph [0060] *<br>----- | 1-15 | |

TECHNICAL FIELDS SEARCHED (IPC)

C07C

The present search report has been drawn up for all claims

1

| Place of search | Date of completion of the search | Examiner |
|---|---|---|
| The Hague | 27 May 2025 | Tabanella, Stefania |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 31 5572

This annex lists the patent family members relating to the patent documents cited in the above-mentioned European search report. The members are as contained in the European Patent Office EDP file on
The European Patent Office is in no way liable for these particulars which are merely given for the purpose of information.

27-05-2025

| Patent document cited in search report | Publication date | Patent family member(s) | Publication date |
|---|---|---|---|
| US 2023020748 A1 | 19-01-2023 | BR 112022006058 A2 | 09-08-2022 |
| | | CA 3155563 A1 | 10-06-2021 |
| | | CN 114729268 A | 08-07-2022 |
| | | EP 4069803 A1 | 12-10-2022 |
| | | FI 20196063 A1 | 31-12-2020 |
| | | JP 2023504849 A | 07-02-2023 |
| | | KR 20220072865 A | 02-06-2022 |
| | | US 2023020748 A1 | 19-01-2023 |
| | | WO 2021110524 A1 | 10-06-2021 |
| EP 2417058 A1 | 15-02-2012 | CN 102459066 A | 16-05-2012 |
| | | EA 201171177 A1 | 30-05-2012 |
| | | EP 2417058 A1 | 15-02-2012 |
| | | ES 2589108 T3 | 10-11-2016 |
| | | JP 5393872 B2 | 22-01-2014 |
| | | JP 2012522721 A | 27-09-2012 |
| | | KR 20120006499 A | 18-01-2012 |
| | | PL 2417058 T3 | 30-12-2016 |
| | | US 2012012471 A1 | 19-01-2012 |
| | | WO 2010115761 A1 | 14-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2534721 A
- WO 2011098525 A
- WO 2014187978 A
- US 20200248321 A
- WO 2015052297 A

### Non-patent literature cited in the description

- Atlas of Zeolite Framework Types. Elsevier, 2007
- **TONG Y. et al.** *Int. J. of Hydrogen Energy*, 2022, vol. 47, 12067-12073
- *CHEMICAL ABSTRACTS*, 513-77-9
- *CHEMICAL ABSTRACTS*, 1306-38-3
- *CHEMICAL ABSTRACTS*, 1314-36-9
- *CHEMICAL ABSTRACTS*, 1313-99-1
- *CHEMICAL ABSTRACTS*, 7440-22-4